# EUROPEAN PATENT APPLICATION

(11) **EP 1 941 878 A1**
(43) Date of publication of application: **09.07.2008**
(21) Application number: 08151101.6
(22) Date of filing: 09.01.2003
(51) Int. Cl.: A61K 31/437, A61K 9/16, A61K 47/14, A61K 47/44, A61K 9/00, A61P 25/20

(54) **Sedative non-benzodiazepine formulations**

(30) Priority: 10.01.2002 US 346613 P
(62) Divisional of application: 03729537.5
(71) Applicant: BIOVAIL LABORATORIES INTERNATIONAL SRL, Collymore Rock Saint Michael (BB)
(72) Inventor: O'Toole, Edel, 3, Dublin (IE); Fogarty, Siobhan, County Dublin (IE)
(74) Representative: Lane, Cathal Michael

(57) **Abstract**

The invention provides for an enhanced absorption pharmaceutical composition comprising a plurality of microparticles, each microparticle comprising at least one sedative non-benzodiazepine, at least one spheronization aid and at least one solubility enhancer. The microparticles of the invention are further incorporated into an oral fast-dispersing dosage form.

## Description

### RELATED APPLICATIONS

This application claims priority from United States provisional patent application No. 60/346,613 filed January 10, 2002.

### FIELD OF THE INVENTION

The present invention relates to enhanced absorption fast-dispersing oral dosage pharmaceutical preparations comprising at least one sedative non-benzodiazepine agent.

### BACKGROUND

Insomnia is defined as difficulty falling asleep, or maintaining sleep, which interferes with a patient's daytime functioning. Insomnia is the most common sleep complaint with a prevalence of 26% to 50% in adult populations *(*Erman ME, Insomnia. Rakel: Conn's Current Therapy. (Ed.) 52nd ed. Saunders Co. 2000*;* Shader RI. Sedative-Hypnotics. Tasman: Psychiatry, 1st ed. Saunders Co. 1997). Although widely prevalent in the general population, it is estimated that only 5% to 20% of patients suffering from insomnia seek help from their clinicians *(*Erman ME, Insomnia. Rakel: Conn's Current Therapy. 52nd ed. Saunders Co. 2000*).* It has been reported that in the United States, 10% to 16% of adults suffer from serious short-term to sub-acute insomnia (i.e., < 6 months in duration) and 9% to 10% complain of serious chronic insomnia (i.e., > 6 months in duration) *(*Insomnia. Goetz:Textbook of Clinical Neurology. 1st ed. Saunders Co. 1999).

Benzodiazepines have been the mainstay of therapy for insomnia and are available as short, intermediate or long-acting hypnotic agents. When used for a short period of time, the benzodiazepines have been useful in treating insomnia. However, the benzodiazepines pose potential problems such as altering sleep architecture, rebound insomnia when discontinued, possible hangover effects and abuse potential, as well as development of tolerance to the drug. Benzodiazepines act non-selectively on benzodiazepine₁ (omega₁) and benzodiazepine₂ (omega₂) receptors, which may explain their interference with memory, cognition and psychomotor function.

The development of selective benzodiazepine₁ receptor agonists has produced two currently available compounds, Zolpidem (Ambien^{®}, Searle and Co.) and Zaleplon (Sonata^{®}, Wyeth-Ayerst Co.). Zolpidem and Zaleplon are sedative non-benzodiazepine agents that act selectively on benzodiazepine (BZ₁) receptors. By virtue of their short half-life, it is thought that these agents should prevent patients from experiencing benzodiazepine₂ receptor effects involving memory, cognition and psychomotor function. In the literature, neither Zolpidem nor Zaleplon is reported to affect sleep architecture, as do the benzodiazepines.

Zolpidem is an imidazopyridine that binds selectively and potently to the BZ₁ receptor. It does not produce muscle-relaxant or anticonvulsant effects at doses employed for sleep. It has been demonstrated to reduce sleep latency, increase sleep duration, and reduce nighttime awakenings. Zolpidem's half-life is approximately 2.5 hours. Metabolism decreases with age, resulting in the use of doses 50% lower in the elderly. Zolpidem has the advantage that it preserves stage III and IV sleep and has less disruption of REM (Rapid Eye Movement) sleep. Zolpidem is poorly soluble in aqueous media.

There is a known food effect on the pharmacokinetics of zolpidem tartrate. A food-effect study in 30 healthy male volunteers compared the pharmacokinetics of zolpidem tartrate 10mg when administered while fasting or 20 minutes after a meal. Results demonstrated that with food, mean AUC and Cₘₐₓ were decreased by 15% and 25% respectively, while mean Tₘₐₓ was prolonged by 60% (from 1.4 to 2.2 hours). The half-life remained unchanged. These results suggest that, for faster sleep onset, zolpidem tartrate should be administered with or immediately after a meal *(*PDR, 54 Edition, 20005*).*

In one study of eight elderly subjects (>70 years), the means for Cₘₐₓ, T_{½}, and AUC significantly increased by 50% (255 vs. 284 ng/ml), 32% (2.2 vs. 2.9 hours), and 64% (955 vs. 1,562 ng/hr/ml), respectively, as compared to younger adults (20 to 40 years) following a single 20 mg oral zolpidem dose. Zolpidem tartrate did not accumulate in elderly subjects following nightly oral dosing or 10mg for 1 week *(*PDR, 1999).

Zaleplon is a pyrazolopyrimidine derivative that is selective for the BZ₁ receptor but is more weakly bound to the receptor than is Zolpidem. Onset of effect is reported to be slightly more rapid than that for Zolpidem. The half-life is about one hour and is not affected by aging. Zaleplon is not recommended for sleep maintenance. Zaleplon is poorly soluble in aqueous media.

Thus, the difference between Zolpidem and Zaleplon, currently marketed as Ambien® and Sonata® respectively, is that while Sonata® brings on sleep more rapidly than Ambien®, Sonata® acts for a shorter period of time. Ambien®, on the other hand, is more useful for people who have trouble staying asleep all night. Accordingly, it seems that it would be advantageous, given that Zolpidem has far fewer to no nighttime awakening episodes compared to Zaleplon, to develop a dosage form of Zolpidem, whereby the Zolpidem is absorbed into the blood stream faster than that currently possible with Ambien®. Such a dosage form of Zolpidem would bring on sleep much more rapidly than Ambien®, but act just as long as Ambien® and reduce or eliminate the number of nighttime awakenings currently seen with Sonata®.

Oral administration of drugs is the most popular route due to ease of ingestion, pain avoidance, versatility (to accommodate various types of drug candidates), and most importantly, patient compliance. Also, solid oral delivery systems do not require sterile conditions and are, therefore, less expensive to manufacture. Several novel technologies for oral delivery have recently become available to address the physicochemical and pharmacokinetic characteristics of drugs, while improving patient compliance.

The novel technology of oral fast-dispersing dosage forms is known as fast dissolve, rapid dissolve, rapid melt and quick disintegrating tablets. However, the function and concept of all these dosage forms are similar. By definition, a solid dosage form that dissolves or disintegrates quickly in the oral cavity, resulting in solution or suspension without the need for the administration of water is known as oral fast-dispersing dosage form. Several methods are available for the preparation of oral fast-dispersing dosage forms. These include, modified tableting systems, floss, or Shearform^{™} formation by application of centrifugal force and controlled temperature, and freeze-drying. The inclusion of saccharides seems to be the basis for most of these technologies. The choice of material(s) depends on their rapid dissolution in water, sweet taste, low viscosity to provide 'smooth melt feeling', and compressibility.

Current manufactures of oral fast-dispersing dosage forms include, in addition to the assignee of the subject application, Cima Labs, Prographarm/Ethypharm, R.P. Scherer, and Yamanouchi-Shaklee. All of these manufacturers market different types of rapidly dissolving solid oral dosage forms.

Cima Labs markets ORASOLV^{™}, which is an effervescent direct compression tablet purportedly having an oral dissolution time of 5 to 30 seconds, and DURASOLV^{™,} which is a direct compression tablet having a taste-masked active agent and a purported oral dissolution time of 15 to 45 seconds. Cima's U.S. patent No. 5,607,697 describes a solid dosage form consisting of coated microparticles that disintegrate in the mouth. ORASOLV^{®} is a direct compression technology, which utilizes effervescence material and taste-masked active ingredients, and requires only conventional manufacturing equipment. The inclusion of effervescence causes the dosage form to quickly disintegrate following contact with water or saliva. By definition, the effervescence material is a chemical reaction between an organic acid (citric acid, fumaric acid or maleic acid) and a base (sodium bicarbonate, potassium bicarbonate or magnesium bicarbonate), thereby resulting in the generation of carbon dioxide. The concept of effervescence is a well-known formulation art utilized in several dosage forms. However, Cima's technology uses this concept in a modified fashion to achieve fast-disintegrating dosage forms.

The microparticles are prepared by a novel technique involving the dispersion of the active ingredient into a suitable polymer dispersion together with other excipients such as mannitol and magnesium oxide. The microparticle core has a pharmaceutical agent and one or more sweet-tasting compounds having a negative heat of solution selected from mannitol, sorbitol, a mixture of an artificial sweetener and menthol, a mixture of sugar and menthol, and methyl salicylate. The microparticle is coated, at least partially, with material that retards dissolution in the mouth and masks the taste of the pharmaceutical agent. The microparticles are compressed to form a tablet. Other excipients can also be added to the tablet formation. WO 98/46215, assigned to Cima Labs, is directed to a hard, compressed, fast melt formulation having an active ingredient and a matrix of at least a non-direct compression filler and lubricant. A non-direct compression filler is typically not free flowing, in contrast to a direct compression (DC grade) filler, and usually requires additional processing to form free-flowing granules. Cima Labs also has U.S. patents directed to effervescent dosage forms (U.S. Pat. Nos. 5,503,846, 5,223,264, and 5,178,878) and tableting aids for rapidly dissolving dosage forms (U.S. Pat. Nos. 5,401,513 and 5,219,574), and an international patent application directed to rapidly dissolving dosage forms for water-soluble drugs (WO 98/14179).

Ethypharm markets FLASHTAB^{®}, a fast melt tablet having a disintegrating agent such as carboxymethyl cellulose, a swelling agent such as modified starch, and a taste-masked active agent. The tablets have a purported oral disintegrating time of less than one minute (U.S. Pat. No. 5,464,632).

ZYDIS^{®} is marketed by R. P. Scherer Corp. ZYDIS^{®} is a freeze-dried tablet having an oral dissolution time of 2 to 5 seconds. Lyophilized tablets are costly to manufacture and difficult to package because of the tablets sensitivity to moisture and temperature. U.S. Pat. No. 4,642,903 (R. P. Scherer Corp.) refers to a fast melt dosage formulation prepared by dispersing a gas throughout a solution or suspension to be freeze-dried. U.S. Pat. No. 5,188,825 (R. P. Scherer Corp.) refers to freeze-dried dosage forms prepared by bonding or complexing a water-soluble active agent to or with an ion exchange resin to form a substantially water insoluble complex, which is then mixed with an appropriate carrier and freeze dried. U.S. Pat. No. 5,613,023 (R. P. Scherer Corp.) refers to freeze-dried drug dosage forms made by adding xanthum gum to a suspension of gelatin and active agent. U.S. Pat. No. 5,827,541 (R. P. Scherer Corp.) discloses a process for preparing solid pharmaceutical dosage forms of hydrophobic substances. The process involves freeze-drying a dispersion containing a hydrophobic active ingredient and a surfactant, in a non-aqueous phase; and a carrier material, in an aqueous phase.

The WOWTAB^{®}, by Yamanouchi Pharma Technologies, features a tablet of sufficient hardness to maintain the physical characteristics of the dosage form during production and distribution, until it comes into contact with moisture, such as saliva in the mouth. The WOWTAB^{®} is a tablet having a combination of a low moldability and a high moldability saccharide. U.S. patents covering this technology include U.S. Pat. No. 5,576,014 and U.S. Pat. No. 5,446,464.

Other companies owning oral fast-dispersing dosage forms include Janssen Pharmaceutica (QUICKSOLV^{®}). U.S. patents assigned to Janssen Pharmaceutica describe rapidly dissolving tablets having two polypeptide (or gelatin) components and a bulking agent, wherein the two components have a net charge of the same sign, and the first component is more soluble in aqueous solution than the second component. See U.S. Pat. No. 5,807,576, 5,635,210, 5,595,761, 5,587,180, and 5,776,491.

L.A.B. Pharmaceutical Research owns U.S. patents directed to effervescent-based rapidly dissolving formulations having an effervescent couple of an effervescent acid and an effervescent base (U.S. Pat. Nos. 5,807,578 and 5,807,577).

Schering Corporation has technology relating to buccal tablets having an active agent, an excipient (which can be a surfactant) or at least one of sucrose, lactose, or sorbitol, and either magnesium stearate or sodium dodecyl sulfate (U.S. Pat. Nos. 5,112,616 and 5,073,374).

Laboratoire L. Lafon owns technology directed to conventional dosage forms made by lyophilization of an oil-in-water emulsion in which at least one of the two phases contains a surfactant (U.S. Pat. No. 4,616,047). For this type of formulation, the active ingredient is maintained in a frozen suspension state and is tableted without micronization or compression, as such process could damage the active agent.

Takeda Chemicals Inc., Ltd. owns technology directed to a method of making a fast dissolving tablet in which an active agent and a moistened, soluble carbohydrate are compression molded into a tablet, followed by drying of the tablets.

Biovail Corporation markets FLASHDOSE^{®}, a direct compression tablet containing a processed excipient called SHEARFORM^{®}, SHEARFORM^{®} is a floss type substance of mixed polysaccharides converted to amorphous fibers. U.S. patents describing this technology include U.S. Pat. Nos. 5,871,781, 5,869,098, 5,866,163, 5,851,553, 5,622,719, 5,567,439 and 5,587,172.

One way to provide self-binding flowable formulations is to formulate using SHEARFORM^{®} matrices or flosses. These matrices result when using certain processing techniques, such as that described in U.S. Pat. No. 5,587,172, which is incorporated herein by reference. The patent discusses the use of flash heat techniques to produce sucrose-containing shearform flosses, which are then processed to yield quick-dissolving tablets.

The use of shearform matrices for forming comestible units is described in U.S. Pat. No. 5,895,664. This document discloses a quick dissolving tablet which is formed by: (1) using flash-flow technology to provide a shearform matrix; (2) combining the partially re-crystallized shearform matrix with an additive to form flowable, compactable particulate blends; and (3) compacting the blends at relatively low pressures to produce dosage forms, such as tablets.

Additionally, U.S. Pat. No. 5,851,553 discloses a process an apparatus for making rapidly dissolving forms by flash-flow processing. The patent describes a shearform matrix formed by a flash-flow process, the shearform matrix is combined with an additive, and the matrix is molded to make a unit dosage form.

U.S. Pat. Nos. 5,840,331 and 6,048,541 describe tablet formulations derived from saccharide-based carriers in which the use of a unique combination of feedstock ingredients yields self-binding, flowable matrices and tablet compositions. This combination, which uses a blend of sugar alcohols, i.e., sorbitol and xylitol, is superior to glycerine in providing cohesive properties and flowability.

Shapeable, preferably tabletable, compositions derived from partially hygroscopic matrices containing the sugar alcohols are useful in the presence of tableting aids and crystallization promoters in both high and low-pressure tableting processes. Tablets and other dosage forms, e.g., lozenges, made therefrom rapidly dissolve when placed in the mouth, generally in less than 30 seconds.

The production of microparticles containing active agent(s) is described in U.S. Pat. No. 5,683,720, which is incorporated herein by reference. The patent deals with the use of LIQUIFLASH^{®} processing to spheronize compositions containing one or more active agents.

U.S. Pat. No. 6,165,512, owned by the applicant, provides compositions and shaped oral dosage forms made therefrom having improved properties. Among those properties are improved processability before shaping and enhanced dissolution and taste-masking properties when the dosage forms are used. The compositions of the '512 patent are based on matrices, or flosses, which comprise at least one sugar alcohol, which matrices are generally considered "single floss" or "unifloss" systems. These systems are exemplified by xylitol-containing shearform matrices, or flosses, containing a carrier and two or more sugar alcohols.

Various ingredients, such as coated microparticles containing active agent(s), are added, in suitable amounts, to the compositions of the present invention after the matrices are collected and chopped, but before they are shaped, e.g., by tableting.

Highly useful dosage forms result when microparticles made from compositions containing active agents, solubilizers and spheronization aids are coated with taste-masking agents, then combined with flosses and conventional pharmaceutical ingredients. The resultant tablets enjoy the processing ease associated with the use of glycerine-free flosses and the taste and release properties associated with coated microparticles.

Given that Zolpidem shows very few to no awakening episodes, it would be advantageous to achieve a much more rapid onset of sleep than currently available with Ambien®. One advantageous way of achieving a more rapid onset of sleep with the administration of Zolpidem would be to develop an enhanced absorption oral fast-dispersing dosage form of the drug. The technologies described above are most suitable for water-soluble active agent(s). Zolpidem, however, is a poorly water-soluble drug. The challenge therefore remains to develop an enhanced absorption fast-dispersing oral dosage form of Zolpidem.

### DEFINITIONS

The phrase "oral fast-dispersing dosage form" as used herein is interchangeable with fast dissolve, rapid dissolve, rapid melt, quick disintegrating dosage forms, fast disperse and orally disintegrating tablets, and the like. All such dosage forms are typically in the form of tablets and are adapted to dissolve, disperse or disintegrate rapidly in the oral cavity, resulting in a solution or suspension without the need for the administration of water. Any such dosage form is consistent with the objects of the invention. It is preferred that the dosage form of the invention dissolve, disintegrate or disperse in 50 seconds or less, preferably in 30 seconds or less and most preferably in 20 seconds or less. In the context of the present invention, dissolution relates to water-soluble ingredients only.

As used herein, "enhanced absorption" means a lower T₅₀ with an equal or higher Cₘₐₓ, when compared to the currently marketed sedative non-benzodiazepine agent zolpidem product Ambien^{®}, but having an area under the plasma-concentration time curve (AUC) that is equivalent to Ambien^{®}. Cₘₐₓ is the observed maximum plasma concentration and can be measured after a single-dose or steady-state of the sedative non-benzodiazepine agent for every dose given. Wagner-Nelson deconvolution defines T₅₀ as the time taken for 50% of the drug to be absorbed into the system. The reference for Wagner-Nelson deconvolution can be found in: Gibaldi M., Perrier D. Pharmacokinetics. New York: Marcel Dekker, Inc., 1982. The AUC, or the Area Under the Curve, of the pharmacokinetic profile, signifies the extent of absorption of a drug.

The sedative non-benzodiazepine agent zolpidem as used herein includes the tartrate (also referred to in the art as hemitartrate) of zolpidem or any other pharmaceutically acceptable salt of zolpidem consistent with the objects of the invention.

An effective amount of the sedative non-benzodiazepine agent, preferably zolpidem, is specifically contemplated. By the term "effective amount," it is understood that "a pharmaceutically effective amount" is contemplated. A "pharmaceutically effective amount" is the amount or quantity of the sedative non-benzodiazepine agent, preferably zolpidem, which is sufficient to elicit an appreciable biological response when administered to a patient.

The dosage form of the invention is administered in the evening either in the fed or fasted state. When administered in the fed state, the dosage form of the invention is administered within 30 minutes of the last meal of the day. When administered in the fasted state, the dosage form of the invention is administered at least after 4 hours after the last meal of the day.

### SUMMARY OF THE INVENTION

There is an unmet need for the development of an enhanced absorption fast-dispersing oral dosage form for the treatment of insomnia. Accordingly, this invention relates to an enhanced absorption fast-dispersing oral dosage form comprising at least one sedative non-benzodiazepine agent. The sedative non-benzodiazepine agent is preferably zolpidem.

In one aspect of the invention, there is provided an enhanced absorption pharmaceutical composition comprising a plurality of microparticles, each microparticle comprising at least one sedative non-benzodiazepine agent, at least one spheronization aid and at least one solubility enhancer.

The sedative non-benzodiazepine agent is about 1 % to about 55% by weight of the microparticle, preferably about 12.5% to about 17.5% by weight of the microparticle and most preferably about 15% by weight of the microparticle. The spheronization aid is about 5% to about 85% by weight of the microparticle, preferably about 45% to about 55% by weight of the microparticle and most preferably about 50% by weight of the microparticle. The solubility enhancer(s) comprising the microparticles are in the range of from greater than 0% to about 90%, preferably from about 30% to about 40% by weight and most preferably about 35% by weight of the microparticle. The preferred sedative non-benzodiazepine agent is zolpidem, the preferred spheronization aid is distilled monoglycerides, and the preferred solubility enhancer is a macrogol fatty acid ester with Gelucire 50/13® being the most preferred macrogol fatty acid ester.

The microparticles of the invention can be incorporated into tablets or capsules as uncoated microparticles or microparticles coated with at least one taste-masking coat. When incorporated into tablets, it is preferred that the microparticles be coated with at least one taste-masking coating.

In a preferred embodiment, the tablet incorporating the microparticles of the invention is an oral fast-dispersing tablet adapted to rapidly dissolve in the mouth of a patient.

In another aspect of the invention there is provided an oral fast-dispersing dosage form comprising: (a) microparticles comprising at least one sedative non-benzodiazepine agent, at least one spheronization aid and at least one solubility enhancer, said microparticles coated with at least one taste-masking coating and adapted for enhanced absorption of the sedative non-benzodiazepine agent; and (b) a matrix having enhanced self binding characteristics, wherein said coated microparticles are dispersed within said matrix and said dosage form is adapted to rapidly dissolve in the mouth of a patient. It is preferred that the sedative non-benzodiazepine agent is zolpidem, the spheronization aid is distilled monoglycerides and the solubility enhancer is a macrogol fatty acid ester. It is preferred that that the macrogol fatty acid ester be Gelucire 50/13®. The zolpidem is present in an amount of about 4% by weight of the dosage form, the distilled monoglycerides is present in an amount of about 13.33% by weight of the dosage form, and the macrogol fatty acid ester is present in an amount of about 9.33% by weight of the dosage form. It is preferred that the matrix is a shearform matrix consisting essentially of at least one saccharide carrier and at least two sugar alcohols, comprising sorbitol and about 0.5% to about 25% by weight of xylitol which matrix has been treated with at least one crystallization modifier. The crystallization modifier is preferably Tween 80.

It is preferred that the oral fast dispersing dosage form when administered in the evening to a patient in need of such administration exhibit a blood absorption profile such that after about 0.25 hours at least about 10% of the zolpidem is absorbed, after about 0.5 hours at least about 25% of the zolpidem is absorbed; after about 0.75 hours at least about 35% of the zolpidem is absorbed; after about 1 hour at least about 40% of the zolpidem is absorbed, after about 1.5 hours at least about 50% of the zolpidem is absorbed, in at about 1.75 hours at least 55% of the zolpidem is absorbed, after about 2 hours at least about 60% of the zolpidem is absorbed, after about 4 hours at least about 75% of the zolpidem is absorbed, and after about 6 hours more than about 90% of the zolpidem is absorbed, into the blood stream of the patient in the fed state.

In the fasting state, however, it is preferred that the oral fast dispersing dosage form when administered to a patient in need of such administration in the evening exhibit a blood absorption profile such that after about 0.25 hours at least about 5% of the zolpidem is absorbed, after about 0.5 hours at least about 55% of the zolpidem is absorbed, after about 0.75 hours at least about 75% of the zolpidem is absorbed, after about 1 hour at least about 80% of the zolpidem is absorbed, after about 1.5 hours at least about 85% of the zolpidem is absorbed, after about 2 hours at least about 90% of the zolpidem is absorbed, and after about 4 hours at least about 95% of the zolpidem is absorbed into the blood stream of the patient.

In another aspect of the present invention, the dosage form, provides a Tₘₐₓ for the sedative non-benzodiazepine agent zolpidem ranging from about 0.5 hours to about 6 hours and a Cₘₐₓ ranging from about 42 ng/ml to about 141 ng/ml zolpidem in the blood after administration of the dosage form to a human in the fed state with a mean Tₘₐₓ of about 2.8 hours and a mean Cₘₐₓ of about 82.4 ng/ml zolpidem in the blood after administration of the dosage form.

In another aspect of the present invention, the dosage form, when orally administered in the evening to a fasting patient provides a Tₘₐₓ for the sedative non-benzodiazepine agent zolpidem ranging from about 0.5 hours to about 4 hours and a Cₘₐₓ ranging from about 48 ng/ml to about 189 ng/ml zolpidem in the blood after administration of the dosage form to a human with a mean Tₘₐₓ of about 1.6 hours and a mean Cₘₐₓ of about 112.7 ng/ml zolpidem in the blood after administration of the dosage form to a human.

In another aspect of the present invention, the dosage form, when administered orally to a fed patient, provides a plasma concentration time curve with an AUC₍₀₋ₜ₎ ranging from about 216 ng.hr/ml to about 1352 ng.hr/ml with a mean AUC₍₀₋ₜ₎ of about 633 ng.hr/ml.

In another aspect of the present invention, the dosage form, when administered orally to a fed patient, provides a plasma concentration time curve with an AUC_{(0-infinity)} ranging from about 220 ng.hr/ml to about 1408 ng.hr/ml with a mean AUC_{(0-infinity)} of about 646 ng.hr/ml.

In another aspect of the present invention, the dosage form, when administered orally to a fasting patient, provides a plasma concentration time curve with an AUC₍₀₋ₜ₎ ranging from about 167 ng.hr/ml to about 1764 ng.hr/ml with a mean AUC₍₀₋ₜ₎ of about 688 ng.hr/ml.

In another aspect of the present invention, the dosage form, when administered orally to a fasting patient, provides a plasma concentration time curve with an AUC_{(0-infinity)} ranging from about 170 ng.hr/ml to about 1873 ng.hr/ml with a mean AUC_{(0-infinity)} of about 702 ng.hr/ml.

Accordingly, the advantage of the enhanced absorption fast-dispersing oral dosage form of the invention is that it provides a shorter C₅₀ % absorption in both the fed and fasted patient than the currently marketed oral form of zolpidem (Ambien®), which may translate into a clinical advantage. Further, the taste-masked microparticles can deliver zolpidem through orally dispersible or conventional means.

### BRIEF DESCRIPTION OF THE DRAWINGS

The present invention will be further understood from the following detailed description with references to the following drawings.
**FIG. 1** is a graph illustrating the dissolution profile of uncoated microparticles according to an embodiment of the invention.
**FIG. 2** is a graph illustrating the dissolution profile of coated microparticles according to an embodiment of the invention.
**FIG. 3** is a graph illustrating the dissolution profile of a zolpidem tartrate 10mg FlashDose tablet made according to an embodiment of the invention across a range of pH values.
**FIG. 4** is a graph illustrating the dissolution profile of the prior art 10 mg Ambien® tablet across a range of pH values.
**FIG. 5A** is a graph illustrating the mean in vivo zolpidem plasma concentrations following single-dose zolpidem tartrate 10mg FlashDose tablet made according to an embodiment of the invention administered under fed conditions over a 24-hour period.
**FIG. 5B** is a graph illustrating the differences of the graph in Figure 5A to the prior art 10mg Ambien^{®} tablet administered under fed conditions over a 24-hour period.
**FIG. 5C** is a graph further illustrating the differences in the mean in vivo zolpidem plasma concentrations of Figure 5B during the first 4 hours.
**FIG. 5D** is a graph illustrating the differences in the mean in vivo absorption profiles following single-dose zolpidem tartrate 10mg FlashDose tablet made according to an embodiment and the prior art 10mg Ambien^{®} tablet up to the first hour following administration under fed conditions.
**FIG. 6A** is a graph illustrating the mean in vivo zolpidem plasma concentrations following single-dose zolpidem tartrate 10mg FlashDose tablet made according to an embodiment of the invention administered under fasting conditions over a 24-hour period.
**FIG. 6B** is a graph illustrating the comparison of the graph in Figure 6A and the prior art 10mg Ambien^{®} tablet administered under fasting conditions over a 24-hour period.
**FIG. 6C** is a graph further illustrating the differences in the mean in vivo zolpidem plasma concentrations of Figure 6B during the first 4 hours.
**FIG. 6D** is a graph illustrating the differences in the mean in vivo absorption profiles of the zolpidem tartrate 10mg FlashDose tablet made according to an embodiment and the prior art 10mg Ambien^{®} tablet up to the first hour following administration under fasting conditions.

### DETAILED DESCRIPTION OF THE INVENTION

This invention relates to an enhanced absorption fast-dispersing oral dosage form comprising at least one sedative non-benzodiazepine agent. The sedative non-benzodiazepine agent is preferably zolpidem. The dosage form comprises (a) microparticles comprising at least one sedative non-benzodiazepine agent, preferably zolpidem, at least one solubility enhancer and at least one spheronization aid and (b) a matrix having enhanced self-binding characteristics.

### I. Microparticles

The microparticles of the invention comprise an effective amount of at least one sedative non-benzodiazepine agent, at least one spheronization aid and at least one solubility enhancer. The term "microparticles" as used herein is interchangeable with the terms "microspheres", "spherical particles" and "microcapsules".

The sedative non-benzodiazepine agents(s) used herein can be selected from the group of sedative non-benzodiazepine agents, which include, but are not limited to zolpidem, zaleplon, zoplicone, trazodone, nefazodone, indiplon, esoplicone, chloral hydrate, chloral betaine, mirtazapine, clomethiazole, promethazine, CCD-3693, Co-326, IP-100-9, PPRT-211, SC-72393, TAK-375, and ethychlorvynol. The invention also contemplates any combination of the above sedative-hypnotic therapeutic agents and are not limited to the above listed sedative non-benzodiazepine agents.

Particularly useful sedative non-benzodiazepine agents are those that are sparingly soluble and whose dissolution and release properties in-vivo are enhanced by the solubilizing agents used herein. The most preferred sedative non-benzodiazepine agent is zolpidem. The amount of sedative non-benzodiazepine agent(s), comprising the microparticles is in the range of from about 1 % to about 55%, preferably from about 12.5% to about 17.5% and most preferably about 15% by weight of the microparticle.

Spheronization aid(s) used herein are materials, which help the drug-containing mix to form robust durable spherical particles. Some examples of the preferred materials useful as spheronization aids include, but are not limited to distilled monoglycerides, glyceryl behenate, glyceryl palmitostearate, hydrogenated vegetable oils, sodium lauryl sulfate, polyoxyethylene ethers, cetostearyl alcohol, waxes and wax-like materials. Certain thermo-plastic or thermo-softening polymers may also function as spheronization aids. Some non-limiting examples of such thermo-plastic or thermo-softening polymers include Povidone, cellulose ethers and polyvinylalcohols. Mixtures of spheronization aids can also be used. The most preferred spheronization aid is distilled monoglycerides, as for example DMG-03VF. The spheronization aid(s) comprising the microparticles is in the range of from about 5% to about 85%, preferably from about 45% to about 55% and most preferably about 50% by weight of the microparticle.

Solubility enhancers are surfactants and other materials included in the microparticles to assist in the dissolution of a drug, particularly of poorly soluble drugs. The ability of a surfactant to reduce the solid/liquid interfacial tension will permit fluids to wet the solid more effectively and thus aid the penetration of fluids into the drug-surfactant mass to increase the dissolution and absorption of the drug. Some examples of the preferred materials useful as solubility enhancers include macrogol fatty acid esters, polyethylene glycol, polyethylene glycol derivatives of lipophilic molecules such as polyethylene glycol fatty acid esters, polyethylene glycol fatty alcohol ethers, polymeric surfactant materials containing one or more polyoxyalkylene blocks, such as poloxamers, and other polyoxyethylene/polyoxypropylene copolymers as well as sucrose ethers and esters. Combinations of solubility enhancers can be used. The preferred solubility enhancers are the macrogol fatty acid esters Gelucire 50/13^{®} or Gelucire 44/14^{®} with Gelucire 50/13^{®} being the most preferred. The solubility enhancer(s) comprising the microparticles are in the range of from greater than 0% to about 90%, preferably from about 30% to about 40% by weight and most preferably about 35% by weight of the microparticle.

It is preferred that the microparticles contain only the sedative non-benzodiazepine agent(s), solubilizer(s) and spheronization aid(s). However, other excipients consistent with the objects of the invention may also be used.

The microparticles of the invention are manufactured using the applicant's proprietary CEFORM^{™} (Centrifugally Extruded & Formed Microspheres) technology, which is the simultaneous use of flash heat and centrifugal force, using proprietary designed equipment, to convert dry powder systems into microspheres of uniform size and shape. The microspheres of the invention are prepared by hot-melt encapsulation described in detail in U.S. Pat. Nos. 5,587,172, 5,616,344, and 5,622,719, whose contents are wholly incorporated herein by reference. The process for manufacturing the microparticles of the invention are not limited to the CEFORM^{™} technology, and any other technology resulting in the formation of the microparticles consistent with the objects of the invention may also be used.

The processing of the microparticles is carried out in a continuous fashion, whereby a pre-blend of drug and excipients is fed into a spinning "microsphere head", also termed as an "spheronizing head". The microsphere head, which is a multi-aperture production unit, spins on its axis and is heated by electrical power. The drug and excipient(s) pre-blend is fed into the center of the head with an automated feeder. The material moves, via centrifugal force, to the outer rim where the heaters, located in the rim of the head, heat the material. Microspheres are formed when the molten material exits the head, which are then cooled by convection as they fall to the bottom of the Microsphere Chamber. The product is then collected and stored in suitable product containers. Careful selection of the types and levels of excipient(s) control microparticle properties such as sphericity, surface morphology, and dissolution rate. The advantage of such a process is that microspheres are produced and collected from a dry feedstock without the use of any organic solvents.

Two fundamental approaches are used to produce microspheres: (1) the *encapsulation* process and (2) the *co-melt* process. In the encapsulation approach, the process is conducted below the melting point of the drug. Therefore, the excipients are designed to melt and entrain the drug particles on passing through the apertures to form microspheres. The resulting microspheres contain the drug, in its native state, essentially enveloped by or as an intimate matrix with the resolidified excipients. In the co-melt approach, the process is conducted above the melting point of the drug. In this case, the drug and the excipients melt or become fluid simultaneously upon exposure to the heat. The molten mixture exits the head and forms microspheres, which cool as they fall to the bottom of the collection bin where they are collected.

The microparticles of the invention comprising the sedative non-benzodiazepine agent(s) are manufactured using the encapsulation approach, with at least one spheronizing agent, which also acts as a drug carrier, and at least one solubility enhancer. The encapsulation approach is favored because it is believed that the hydrophilic solubilizer(s) encapsulates the hydrophobic sedative non-benzodiazepine agent(s), thus aiding the solubility of the sedative non-benzodiazepine agent. In the encapsulation technique the excipient(s) which are chosen must a have a lower melting point than the drug with which they will be combined (158.4-159 reference: Merck Index, 12^{th} edition). Therefore the spheronizing process can be performed at lower temperatures, than the melting point of the drug. This eliminates the risk of polymeric interconversion, which can occur when using processing temperatures close to the melting point. The microspheres can also be prepared using other techniques such as fluid bed or melt extrusion, however, the CEFORM^{™} process is the preferred method of manufacturing.

In an alternative embodiment of the invention, it is preferred that the microparticles be coated with at least one coating after the spheronization process to mask the taste of any unpleasant tasting active in the microparticles. Useful coating formulations contain polymeric ingredients as well as excipient(s) conventionally employed in such coatings.

Useful taste-masking coatings can include (meth)acrylate/cellulosic polymers. Ethylcellulose (EC), hydroxypropylcellulose (HPC), hydroxypropylmethylcellulose (HPMC),and polymethacrylate polymers, such as Eudragit RS, Eudragit RL, E 100, and NE30D or mixtures thereof are useful.

The cellulosic coatings are generally applied to the microparticles after spheronization from an organic solvent solution(s). Typical solvents include one or more of acetone, alkyl alcohols (e.g., isopropyl alcohol), and the like. Coating devices used to coat the microparticles of the invention include those conventionally used in pharmaceutical processing, with fluidized bed coating devices being preferred. The coatings applied to the microparticles may contain ingredients other than the cellulosics. Thus, one or more colorants, flavorants, sweeteners, can also be used in the coating formulations.

Colorants used include food, drug and cosmetic colors (FD&C), drug and cosmetic colors (D&C) or external drug and cosmetic colors (Ext. D&C). These colors are dyes, lakes, and certain natural and derived colorants. Useful lakes include dyes absorbed on aluminum hydroxide or other suitable carriers.

Flavorants may be chosen from natural and synthetic flavoring liquids. An illustrative list of such agents includes volatile oils, synthetic flavor oils, flavoring aromatics, oils, liquids, oleoresins and extracts derived from plants, leaves, flowers, fruits, stems and combinations thereof. A non-limiting representative list of these includes citric oils, such as lemon, orange, grape, lime and grapefruit, and fruit essences, including apple, pear, peach, grape, strawberry, raspberry, cherry, plum, pineapple, apricot, or other fruit flavors.

Other useful flavorings include aldehydes and esters, such as benzaldehyde (cherry, almond); citral, i.e., alpha-citral (lemon, lime); neral, i.e., beta-citral (lemon, lime); decanal (orange, lemon); aldehyde C-8 (citrus fruits); aldehyde C-9 (citrus fruits); aldehyde C-12 (citrus fruits); tolyl aldehyde (cherry, almond); 2,6-dimethyloctanal (green fruit); 2-dodenal (citrus mandarin); mixtures thereof and the like.

The sweeteners may be chosen from the following non-limiting list: glucose (corn syrup), dextrose, invert sugar, fructose, and mixtures thereof (when not used as a carrier); saccharin and its various salts, such as sodium salt; dipeptide sweeteners such as aspartame; dihydrochalcone compounds, glycyrrhizin; Steva Rebaudiana (Stevioside); chloro derivatives or sucrose such as sucralose; and sugar alcohols such as sorbitol, mannitol, xylitol, and the like. Also contemplated are hydrogenated starch hydrolysates and the synthetic sweeteners such as 3,6-dihydro-6-methyl-1-1-1,2,3-oxathiazin-4-1-2,2-dioxide, particularly the potassium salt (acesulfame-K), and sodium and calcium salts thereof. The sweeteners may be used alone or in any combination thereof.

The diameter of the uncoated and coated microparticles range from about 150µm in diameter to about 500µm in diameter, preferably from about 200µm to about 300µm and most preferably from about 200µm to about 250µm. Coating levels of about 0% to about 100% are effective, preferably about 15-30% and most preferably about 25%.

### II. Dosage Forms

Due to the spherical nature of the coated and uncoated microparticles of the invention and their robustness, attributed to the high quantity of spheronization aid(s), the microparticles of the invention can be used in a number of different delivery systems including FLASHDOSE^{™} tablets, gelatin capsules, direct compression tablets, buccal tablets and the like.

It is preferred that the coated taste-masked microparticles of the invention be incorporated into the FLASHDOSE^{™} tablet, which is a direct compression tablet containing a processed excipient called SHEARFORM^{™}. SHEARFORM^{™} is a floss type substance of mixed polysaccharides converted to amorphous fibers. The terms "floss" and "matrix" are used interchangeably herein.

The preparation of flosses suitable for use in the present invention is disclosed in U.S. Pat. Nos. 5,622,719, 5,851,553, 5,866,163 all for "Process and Apparatus for Making Rapidly Dissolving Dosage Units and Product Therefrom" and 5,895,664 for "Process for forming quickly dispersing comestible unit and product therefrom", the contents of which are incorporated herein by reference. Preferably, the floss is a "shearform matrix" produced by subjecting a feedstock which contains a sugar carrier to flash-heat processing.

In the flash-heat process, the feedstock is simultaneously subjected to centrifugal force and to a temperature gradient, which raises the temperature of the mass to create an internal flow condition, which permits part of it to move with respect to the rest of the mass. The flowing mass exits through openings provided in the perimeter of a spinning head. The temperature gradient is supplied using heaters or other means which cause the mass' temperature to rise. Centrifugal force in the spinning head flings the internally flowing mass outwardly, so that it reforms as discrete fibers with changed structures.

An apparatus, which produces suitable conditions, is a modified floss-making machine, such as that described in U.S. Pat. No. 5,834,033, entitled "Apparatus for Melt Spinning Feedstock Material having a Flow Restricting Ring". The entire content of that application is hereby incorporated by reference.

Typically, spinning is conducted at temperatures and speeds of about 180 to 250°C and 3,000 to 4,000 rpm, respectively.

Suitable spinner heads include that disclosed in U.S. Pat. No.5,458,823, which contents is hereby incorporated by reference.

Other useful apparatuses or processes that provide similar forces and temperature gradient conditions can be used.

Floss or matrix particles can be chopped using the apparatus discussed in U.S. Pat. No.5,637,326 or another device having a similar function.

The matrices used herein include a carrier, or feedstock material, which comprises at least one material selected from materials which are capable of undergoing the physical and/or chemical changes associated with flash heat processing. Useful carriers include carbohydrates, which become free-form particulates when flash heat processed. Saccharide-based carriers, including saccharides (i.e., sugars), polysaccharides and mixtures thereof can be used.

The feedstocks used in the invention can include carriers chosen from various classes of "sugars". "Sugars" are those substances, which are based on simple crystalline mono- and di-saccharide structures, i.e., based on C₅ and C₆ sugar structures. They can include glucose, sucrose, fructose, lactose, maltose, pentose, arabinose, xylose, ribose, mannose, galactose, sorbose, dextrose and sugar alcohols, such as sorbitol, mannitol, xylitol, maltitol, isomalt, sucralose and the like and mixtures thereof. Sucrose is the preferred sugar.

Useful mixtures of carriers include the sugars listed above along with additional mono- di-, tri- and polysaccharides. Additional saccharides can be used in amounts of up to 50% by weight of the total sugar, preferably up to 30%, most preferably up to 20%.

Optionally, the polysaccharides can be used alone as carriers. Polysaccharide carriers include polydextrose and the like. Polydextrose is a non-sucrose, essentially non-nutritive, carbohydrate substitute. It can be prepared through polymerization of glucose in the presence of polycarboxylic acid catalysts and polyols. Generally, polydextrose is commercially available in three forms: polydextrose A and polydextrose K, which are powdered solids; and polydextrose N supplied as a 70% solution. The contents of U.S. Pat. No. 5,501,858, which discusses polydextrose is incorporated herein by reference.

If other carrier materials are used, they are employed in combination with sugar and not as total replacement therefor. For example, maltodextrins may be employed. Maltodextrins include mixtures of carbohydrates resulting from the hydrolysis of a saccharide. They are solids having a dextrose equivalent (DE) of up to and including 65.

The carrier can also include malto-oligo-saccharides produced by selective hydrolysis of cornstarch. A general description of maltooligo-saccharides useful herein is set forth in U.S. Pat. Nos. 5.347,341 and 5,429,836 both of which are incorporated herein by reference.

Applicants use the following types of matrix systems, which systems are devoid of glycerine.

In the first system, xylitol is added to a mixture of saccharide-based carrier and one or more additional sugar alcohols, with sorbitol being favored as an additional sugar alcohol. The carrier mix is flash-heat processed to provide shearform floss having self-binding properties. Flosses made using sucrose, sorbitol and xylitol have been found to yield particularly effective self-binding properties. They exemplify "single floss" or "unifloss" systems.

The second system makes separate xylitol-containing binder flosses. The binder flosses ("binder portions") are combined with base flosses ("base portions"), which contain a different sugar alcohol and a saccharide. Preferably, the base floss contains sorbitol and sucrose, while the binder floss contains xylitol. These are termed "dual floss" systems.

The ingredients, which increase cohesiveness and give self-binding properties preferably include sugar alcohols, such as sorbitol, xylitol, maltitol, mannitol and mixtures thereof, all of which form flosses. Other sugar alcohols, especially hygroscopic ones, are contemplated.

Xylitol and sorbitol are the preferred sugar alcohols. Effective amounts of xylitol in the flosses are between about 0.5% and 25%, and preferably about 10% by weight. Sorbitol is used in the flosses in amounts of about 0.5% to about 40% by weight.

When sorbitol and xylitol are used, the ratio of sorbitol to xylitol is from about 1:0.1 to about 1:10.

In dual floss systems, about 20% to about 80%, preferably about 34%, of the total floss content is xylitol-containing, or binder, floss. Likewise, the sorbitol containing, or base, floss may be about 20% to about 80% of the total floss. In some "dual floss" embodiments, xylitol-containing flosses are first mixed with active ingredient(s), then mixed with sucrose/sorbitol flosses.

Regardless of the number of flosses, the total floss content preferably includes about 50% to about 85% sucrose, about 5% to about 20% sorbitol and about 5% to about 25% xylitol.

In some cases, flosses are used along with bio-affecting, or active, microspheres in the tableting process. Often, xylitol-containing floss is added to microspheres of one or more active agents first and then non-xylitol-containing floss is added. Typically, the weight ratio of total floss to microspheres is about 1:1. In these instances, about 5% to about 25% of the floss is xylitol.

Whereas prior art shearform matrices conventionally included a liquid binding additive such as glycerine, the present matrices do not. Instead, they get their enhanced cohesiveness, self-binding character and flowability directly from the matrix or feedstock ingredients and the processing used.

The amorphous shearform matrix of the present invention is preferably made from a feedstock, which includes sucrose, sorbitol, and xylitol. As set forth in U.S. Pat. No. 5,869,098, entitled "Fast Dissolving Comestible Units Formed under High Speed/High Pressure Conditions", these compositions promote recrystallization and tableting of the matrix-containing mixes to a level sufficient to provide particulate flowability for use in high speed and high pressure tableting equipment.

The compositions to be processed into comestible units, or tablets, can contain conventional additives. Conventional quantities of these additives may be incorporated into one or more of the matrices or may be mixed therewith prior to tableting. Useful amounts of conventional additives range from about 0.01 % to about 80% by weight, based on the weight of the matrices or formulations in which they are used. The quantities may vary from these amounts, depending on the functions of the additives and the characteristics desired in the matrices and/or the final tablet compositions.

Conventional tableting aids may be selected from a wide variety of materials such as lubricants, glidants, anti-caking agents and flow agents. For example, lubricants such as adipic acid, magnesium stearate, calcium stearate, zinc stearate, hydrogenated vegetable oils, sodium chloride, sterotex, polyoxyethylene, glyceryl monostearate, talc, polyethylene glycol, sodium benzoate, sodium lauryl sulfate, magnesium lauryl sulfate, sodium stearyl fumarate, light mineral oil and the like may be employed, with sodium stearyl fumarate preferred. Waxy fatty acid esters, such as glyceryl behenate, sold as "Compritol^{™}" products, can be used. Other useful commercial lubricants include "Stear-O-Wet^{™}" and "Myvatex^{™} TL". Mixtures are operable. Lubricants are used in amounts ranging from about 0% to about 10%, with about 0.01 % to about 5.0% typically used.

Glidants such as starch, talc; lactose, stearates, dibasic calcium phosphate, magnesium carbonate, magnesium oxide, calcium silicate, Cabosil^{™}, Syloid^{™}, and silicon dioxide aerogels may be employed. Glidants are present in amounts ranging from about 0% to about 20%, with amounts of about 0.1 % to about 5.0% being typical. Lactose, which may be a glidant or filler, can be added to the chopped floss at about 2% concentration to inhibit clumping.

The preformed matrices produced in accordance herewith may be rendered more crystalline by one or more of the following crystallizing techniques. The nature of the matrix feedstock determines whether the matrix is re-crystallized after it is formed. Nonetheless, "crystallization" and "recrystallization" are used interchangeably herein.

One technique for recrystallizing involves the use of crystallization enhancers. These are used after the floss has been formed, but before the floss-containing composition is tableted. Suitable crystallization enhancers include ethanol, polyvinyl-pyrrolidone, water (e.g. moisture), glycerine, radiant energy (e.g., microwaves) and the like, with combinations being useful. When they are physical materials, typical amounts of these enhancers range from about 0.01 % to about 10.0% by weight of the tablet composition.

Another technique relates to the use of crystallization modifiers. These crystallization modifiers are floss ingredients, used at levels of about 0.01 % to about 20.0% by weight of the floss.

Surfactants are preferred crystallization modifiers. Other materials, which are non-saccharide hydrophilic organic materials may also be used. Useful modifiers preferably have a hydrophilic to lipid balance (HLB) of about 6 or more. Such materials include, without limitation, anionic, cationic, and zwitterionic surfactants as well as neutral materials with suitable HLB values. Hydrophilic materials having polyethylene oxide linkages are effective. Those with molecular weights of at least about 200, preferably at least 400, are highly useful.

Crystallization modifiers useful herein include: lecithin, polyethylene glycol (PEG), propylene glycol (PPG), dextrose, the SPANS^{®} and TWEENS^{®} which are commercially available from ICI America, and the surface active agents known as "Carbowax^{®}". Generally, the polyoxyethylene sorbitan fatty acid esters called TWEEN^{®}s, or combinations of such modifiers are used. Crystallization modifiers are usually incorporated into matrices in amounts of between about 0% and 10%.

Optionally, the matrices are allowed to re-crystallize, with or without added crystallization modifiers, either before or after they are combined with the non-matrix component(s), e.g., the bio-affecting additive(s). When recrystallization occurs before tableting, the recrystallization level of the matrix generally reaches at least about 10%. The use of such partially re-crystallized matrices leads to compositions that are free flowing and tabletable using conventional machines. U.S. Pat. No. 5,597,416 describes a process for recrystallizing in the presence of additives.

Methods for effecting the recrystallization of the matrices include: use of Tween^{®} 80 or other crystallization modifier(s) in the matrix premix; aging of the matrix for up to several weeks, contacting the matrix with sufficient moisture and heat to induce crystallization, and treating the floss or the floss-containing composition with ethanol or another crystallization enhancer. Combinations of these may be used.

When a surfactant, such as a Tween^{®} is used, about 0.001 % to about 1.00% is included in the floss preblend as a crystallization modifier. Following preblending, the formulations are processed into flosses, then chopped and used, with or without additives, to make tablets. Mixtures of surfactants can be used.

Aging may be used to re-crystallize the matrix or floss. The aging process involves a two-step process. First, the matrix, which typically contains at least one crystallization modifier, is formed, chopped and allowed to stand in closed or sealed containers without fluidization or other agitation under ambient conditions, e.g., at room temperature and atmospheric pressure, for up to several days, preferably for about 1 to about 3 days. Later, the matrix is mixed, and optionally further chopped, with one or more other ingredients. The mix is then aged by allowing it to stand for an additional period of about 1 to about 3 days. Generally, the two-step aging process takes a total of about one week, with periods of about 4 to about 5 days being typical.

The flosses may also be re-crystallized by subjecting them to increased heat and moisture. This process is similar to aging, but involves shorter periods of time. Using a fluidized bed apparatus or other suitable device, chopped floss is fluidized while heating, at ambient humidity and pressure, to temperatures of about 25°C. to about 50°C. Typically, the temperature is monitored to minimize clumping of floss particles during this operation. If any clumping occurs, the floss particles must be sieved before being further processed into tablets. Heating times of about 5 to about 30 minutes are typical.

When ethanol is used as a crystallization enhancer, it is used in amounts, based upon the weight of the matrix, of about 0.1% to about 10%, with amounts of about 0.5% to about 8.0% being very effective. The preformed matrix is contacted with ethanol. Excess ethanol is evaporated by drying for about an hour at about 85° F. to about 100° F., with 95° F. being highly useful. The drying step is carried out using tray drying, a jacketed mixer or other suitable method. Following ethanol treatment, the matrix becomes partially re-crystallized on standing for a period ranging from about a few hours up to several weeks. When the floss is about 10% to about 30% re-crystallized, it is tableted after blending with other ingredients. The tableting compositions flow readily and are cohesive.

Re-crystallization of the matrix may take place in the presence of one or more bio-affecting agents or other additives.

Re-crystallization of the matrix can be monitored by measuring the transmittance of polarized light therethrough or by the use of a scanning electron microscope. Amorphous floss or shearform matrix does not transmit polarized light and appears black in the light microscope when viewed with polarized light. Using bright field microscopy or the scanning electron microscope, the surface of the floss appears very smooth. In this condition, it is 0% re-crystallized. That is, the floss is 100% amorphous.

Re-crystallization of amorphous matrix starts at the surface of the mass and can be modified, e.g., accelerated, by the presence of crystallization modifiers, as well as moisture. When TWEEN^{®}s assist the re-crystallization, initiation of re-crystallization is evidenced by a birefringence observed on the surface of the shearform matrix (floss) as viewed with polarized light. There are faint points of light riddled throughout the matrix surface. When birefringence appears, re-crystallization has begun. At this stage, re-crystallization is between about 1% and about 5%.

As re-crystallization proceeds, the birefringence on the surface of the matrix grows continually stronger and appears brighter. The points of light grow in size, number and intensity, seeming to almost connect. Using bright field or scanning electron microscopy, the surface of the matrix appears wrinkled. At this point, about 5 to 10% recrystallization has occurred.

Surfactant (e.g., TWEEN^{®} 80) droplets become entrapped within the matrix. These droplets are obscured as re-crystallization proceeds. As long as they are visible, the floss is generally not more than about 10% to about 20% re-crystallized. When they are no longer observable, the extent of re-crystallization is no more than about 50%.

The re-crystallization of the matrix results in reduction of the total volume of material. Ordered assays of molecules take up less space than disordered arrays. Since recrystallization begins at the surface of the floss, a crust is formed which maintains the size and shape of the floss. There is an increase in the total free volume space within the floss as recrystallization nears completion, which manifests itself as a void inside the floss. This is evidenced by a darkened central cavity in light microscopy and a hollow interior in scanning electron microscopy. At this stage, the floss is believed to be about 50% to about 75% re-crystallized.

The intensity of transmitted polarized light increases as the floss becomes more crystalline. The polarized light can be measured by a photon detector and assigned a value against calculated standards on a gray-scale.

The final observable event in the recrystallization of floss is the appearance of fine, "cat whisker-like" needles and tiny blades, which grow and project from the surface of the floss. These crystals, believed to be sorbitol (cat whiskers) and xylitol (blades), literally cover the floss like a blanket of fuzz. These features can be easily recognized by both light and electron microscopes. Their appearance indicates the final stage of recrystallization. The floss is now about 100% re-crystallized, i.e., substantially non-amorphous.

The matrix portions of the tabletable composition are typically formed via flash-heat processing into floss. The floss strands are macerated or chopped into rods for further processing. Rods of chopped floss have lengths of about 50µm to about 500µm.

Other ingredients, which may be included are fillers and other conventional tablet additives. Additional fragrances, dyes, flavors, sweeteners (both artificial and natural) may also be included, if necessary even though the microspheres to be incorporated into the floss are already taste-masked.

For example, fillers may be used to increase the bulk of the tablet. Some of the commonly used fillers are calcium sulfate, both di- and tri-basic; starch; calcium carbonate; microcrystalline cellulose; modified starches, lactose, sucrose; mannitol and sorbitol. Direct compression fillers may replace shearform for the same function.

If necessary, additional flavorings, sweeteners, dyes and fragrances to be added to the floss may be chosen from the non-limiting lists described above.

Effervescent disintegration agent(s) may also be added. The positive organoleptic sensation achieved by the effervescent action in the mouth, as well as the texture, speed and sensation of disintegration, aid in masking undesirable flavor notes.

"Effervescent" refers to those agents, which evolve gas. The gas- or bubble-generating action is often the result of the reaction of a soluble acid source and a carbonate source. The reaction of these two general classes of compounds produces carbon dioxide gas upon contact with water in saliva. Useful acids include: citric, tartaric, malic, fuimaric, adipic, succinic and acid salts and anhydrides thereof. Acid salts may also include sodium dihydrogen phosphate, disodium dihydrogen pyrophosphate, acid citrate salts and sodium acid sulfite. While the food acids can be those indicated above, acid anhydrides of the above-described acids may also be used. Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate. Mixtures of various acids and carbonate sources, as well as other sources of effervescence, can be used.

The effervescent agent can be included in at least three different ways. The first method includes incorporating the entire effervescent agent in the feedstock, which is used to form the shearform product. The second involves adding the agent to an already formed shearform matrix. The third method incorporates one portion of the agent in the shearform matrix and adds another portion after formation of the matrix material. The artisan can determine the best way to use the agent for its effervescent properties.

Other ingredients include binders, which contribute to the ease of formation and general quality of the tablet. Binders include starches, pregelatinized starches, gelatin, polyvinylpyrrolidone, methylcellulose, sodium carboxymethylcellulose, ethylcellulose, polyacrylamides, polyvinyloxoazolidone and polyvinylalcohols.

The following non-limiting examples illustrate the invention:

### EXAMPLE 1

### Uncoated Microparticles

The following formulation is prepared:

| **Ingredients** | **Amount (%)** |
|---|---|
| Zolpidem tartrate | 15 |
| Macrogol fatty acid ester^{a} | 35 |
| Distilled Monoglycerides^{b} (DMG-03VF) | 50 |
| Total | 100 |

| | |
|---|---|
| a - Gelucire 50/13^{®} b - Myvaplex^{®} | |

Each of the weighed components are transferred into the Littleford FM130 mixer in the order specified below.
1. 1/2 of Distilled monogylceride (DMG-03VF),
2. Zolpidem Tartrate,
3. Milled Gelucire® 50/31
4. Remainder of Distilled monogylceride ( DMG-03VF) Materials are blended for 10mins in total. 5 minutes with Choppers off, 5 minutes with Choppers on. The plow speed is 60Hz.

The above blend was spheronized using the following process parameters. The process called for a % power input of about 22% to about 25% and a head speed of about 45Hz. The process temperature of the microparticle blend was exposed to during the spheronization was about 92.8°C to about 133.6°C.

Samples of the microparticles were taken from the beginning, middle, and end of the spheronizing process to show uniformity. Dissolution profiles are immediate and meet the NLT 80% (Q) at the 30-minute time-point. Assay values meet specification indicating that the microparticles contain a uniform amount of drug.

In process samples were also taken during the screening step. All assay values are within target values and dissolution results are consistent. P.S.A. data is report value but the D₅₀ is in the desired range of 200µm-300µm. The microparticle morphology was examined under a polarized light microscope and was reported as spherical and uniform in shape. Thus, the microparticles were deemed acceptable for coating and were brought forward to the next stage.

The dissolution profile of the above microparticles is determined under the following dissolution conditions:
Medium: 900 ml, pH 5.8 phosphate buffer.
Method: USP Apparatus II at 50 rpm at 37°C ± 0.5°C.

The results are presented below as a % release of the total zolpidem in the microparticles:

| **Time (min)** | **Mean (%)** | **Std. Dev. (%)** | **Min. (%)** | **Max. (%)** |
|---|---|---|---|---|
| 0 | 0 | 0 | 0 | 0 |
| 5 | 91 | 1 | 90 | 92 |
| 10 | 98 | 1 | 98 | 99 |
| 15 | 100 | 1 | 99 | 100 |
| 30 | 99 | 1 | 99 | 100 |
| 60 | 99 | 1 | 99 | 100 |

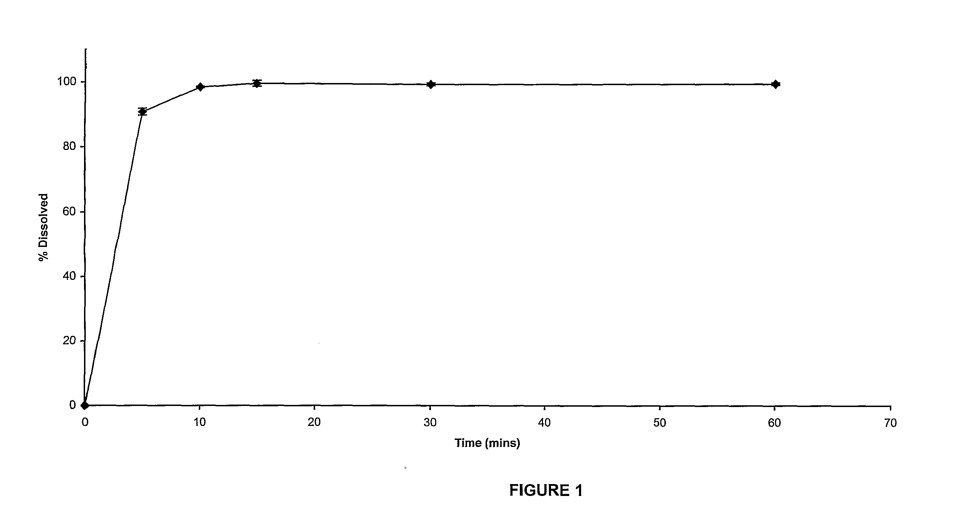

The dissolution profile of the uncoated microparticles prepared as in Example 1 is shown in FIG.1.

### EXAMPLE 2

### Coated Microparticles

The microparticles are reproduced according to the same manufacturing process described above. The microparticles are then coated for taste masking with a coating solution containing a 60:30:10 ratio of NE30D:Talc:HPMC (Methocel^{®}).

The coating consists of a Eudragit NE30D / Talc / HPMC system. Eudragit NE30D is the main component of this system, which provides the barrier layer for successfully masking the taste of the drug and maintaining the in-vitro dissolution within the specified ranges. Eudragit NE30D is a neutral co-polymer of ethylacrylate and methylmethacrylate. It is supplied as a 30% aqueous dispersion. It is a pH-independent, permeable polymer. Thus, films produced from this polymer are insoluble in water, but allow pH-independent drug release.

Hydroxy Propylmethycellulose (HPMC) is a substituted polymer of the natural carbohydrate cellulose. HPMC is very soluble in water and its hydrophilic nature affords a degree of permeability to the coating system necessary for the release of the drug from the core. Talc is a fine white crystalline powder. It is a purified, hydrated magnesium silicate and it acts as a glidant in coating solution formulations. Talc is added to this coating system to aid processing due to the susceptibility of the microparticles to agglomerate. The final formulation consists of a NE30D:Talc:HPMC ratio of 60:30:10. The solid content of this solution was 20.29% w/w, which was made up with purified water.

The solution was prepared by mixing the Talc into the water using a suitable mixer. Once the Talc and water is mixed it is processed through a Debee 2000 homogenizer at a pressure of 40,000PSI.

Once the Talc/water slurry has been homogenized, the homogenate is replaced under the mixer and while stirring slowly the Methocel is added to the homogenate and allowed to mix until the Methocel E5 is dissolved. The resulting suspension is then cooled.

The Eudragit^{®} NE30D suspension is next screened from the product container through a 60-mesh screen into the final container. The Talc/Methocel suspension is also screened through a 60-mesh screen before it is added to the Eudragit^{®} NE30D Suspension.

Coating of the microparticles is carried out in a Glatt GPCG-60 with a wurster size of 18" and a 60mesh bottom screen. The parameters are set as indicated by the table below. The parameters are adjusted during the coating procedure to ensure adequate fluidization, minimize agglomeration and maintain a product temperature of 24-32°C.

| | **Units of Measurement** | **Initial setting** |
|---|---|---|
| Total air volume | cfm | 1200-2650 |
| Process air volume | cfm | 600-900 |
| Inlet air temperature | °C | 25-45 |
| Inlet Dew point temperature | °C | 5-15 |
| Filter Shake duration | Seconds | 4-10 |
| Filter shake interval | Seconds | 10-30 |
| Atomization Air pressure | Bar | 2.7-4.0 |
| Atomization Purge pressure | Bar | 0.8-1.2 |

The initial flow rate for the solution is 70 (+/- 30) g/min. The flow rate increases as the process continues. The coating process is continued until the target weight gain is achieved. At this point the spraying process is terminated and the drying can commence.

Once drying is completed the microparticles are removed form the Glatt chamber and are screened between 150-425um screens.

The dissolution profile of the above-coated microparticles is determined under the following dissolution conditions:
Medium: 900 ml, pH 5.8 phosphate buffer.
Method: USP Apparatus II at 50 rpm at 37°C ± 0.5°C.

The results are presented below as a % release of the total zolpidem in the microparticles:

| **Time (min)** | **Mean (%)** | **Std. Dev. (%)** | **Min. (%)** | **Max. (%)** |
|---|---|---|---|---|
| 0 | 0 | 0 | 0 | 0 |
| 5 | 72 | 1 | 69 | 73 |
| 10 | 82 | 1 | 81 | 83 |
| 15 | 89 | 1 | 88 | 89 |
| 30 | 97 | 1 | 96 | 97 |
| 60 | 101 | 1 | 100 | 101 |

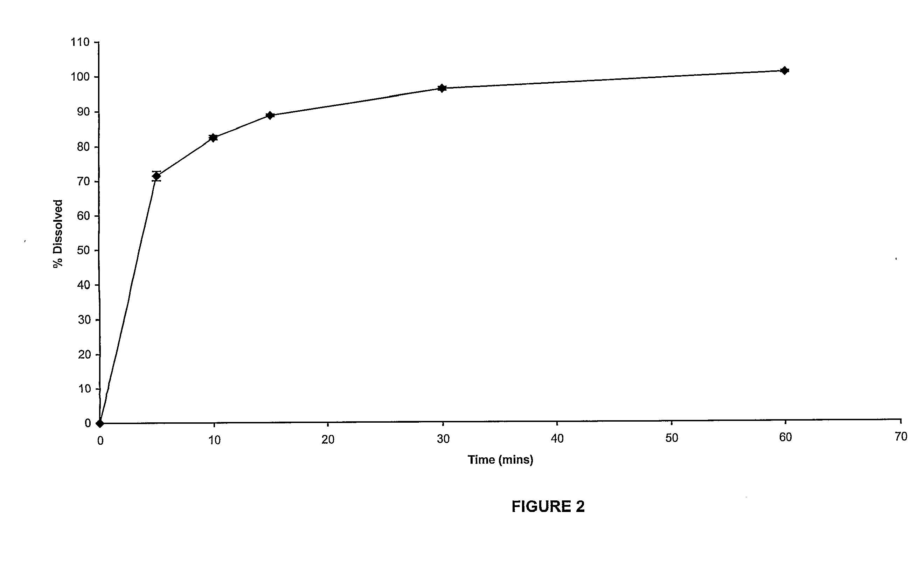

The dissolution profile of the coated microparticles prepared as in Example 2 is shown in FIG.2

### EXAMPLE 3

### Floss/Matrix

A preblend of about 78.25% sucrose, about 11 % sorbitol, 10% xylitol and 0.75% TWEEN (Polysorbate) 80 was prepared.

The floss preblend was processed using 5" crown head disclosed in U.S. Pat. No. 5,854,344 at a temperature of 250°C and a rotational speed of 60 Hz (3600 rpm). The floss collected was chopped in the Littleford FKM600 mixer with 2% lactose (2% w/w of the floss) for 2 minutes at 100 rpm with the choppers on. 200 proof ethanol (0.5% based on weight of the floss) was sprayed on the chopped floss and mixed. The floss was then dried at 45°C for 90 minutes with intermittent mixing. The dried floss was screened through a 20-mesh screen.

### EXAMPLE 4

### Flash Dose Tablets

The coated microparticles as prepared in Example 2 and the floss as prepared in Example 3 were used in the following tablet composition:

| **Tablet Component** | **%w/w of 250 mg Tablet** | **Component Weight (mg)** |
|---|---|---|
| Zolpidem Tartrate | 4.00 | 10.00 |
| Distilled Monoglycerides (DMG-03VF)^{a} | 13.33 | 33.33 |
| Macrogol fatty acid ester^{b} | 9.33 | 23.33 |
| Eudragit NE30D | 4.00 | 10.00 |
| Methocel E5 | 0.67 | 1.67 |
| Talc | 2.00 | 5.00 |
| Floss | 61.37 | 153.43 |
| Silicon dioxide^{c} | 0.50 | 1.25 |
| Sodium Stearyl Fumarated | 1.00 | 2.5 |
| Intense Peppermint Flavor | 1.5 | 3.75 |
| FD&C Blue No.2 | 0.20 | 0.50 |
| NutraSweet^{®} | 0.30 | 0.75 |
| Acesulfame K | 0.50 | 1.25 |
| Natural Debittering flavor | 1.0 | 2.50 |
| Magnasweet^{®} 100 | 0.30 | 0.75 |
| Total | 100 | 250 |

| | | |
|---|---|---|
| a - Myvaplex^{®} b - Gelucire 50/13^{®} c - Syloid^{®} 244FP d - PRUV^{®} | | |

Each of the weighed components is transferred into the Littleford FM130 mixer in the order specified below.
1. ½ of 1.0% ethanol treated floss;
2. All of the Zolpidem Tartrate Taste masked microparticles;
3. Remainder of 1.0% ethanol treated floss
   The above materials are blended for 5 minutes with Choppers off. The plow speed is 45Hz. The following components are next added in the following order:
4. All of the NutraSweet^{®};
5. All off the Acesulfame potassium;
6. All of the Magnasweet^{®} 100;
7. All of the Natural Debittering flavor;
8. All of the Syloid^{®} 244FP;
9. All of the Intense Peppermint flavor;
   The above materials are blended for 5 minutes with Choppers off. The plow speed is 45Hz. The following component is next added and blended with Choppers off for a further 2 minutes. The plow speed is 45 Hz.:
10. All of the PRUV^{®} (-40 mesh);
   Finally, the following component is added and blended for a further of 6 min, with Choppers off. The plow speed is 45 Hz.:
11. All of the FD&C Blue color #2.
   The total tablet blend time is therefore 18 minutes. The blend is subsequently compressed into tablets.

The resulting FlashDose™ tablets formed have a typical hardness value of about 7N to about 13N and a typical tablet thickness of about 4.5 mm.

The dissolution profile of the above FlashDose™ tablet is determined under the following dissolution conditions:
Medium: 900 ml 0.1 N HCl, 900 ml pH 4.5 acetate buffer, 900 ml pH 5.8 phosphate buffer, 900 ml pH 6.8 buffer
Method: USP Apparatus II at 50 rpm at 37°C ± 0.5°C

The FlashDose™ tablet produced the following dissolution profiles at the various pH values specified in the table below:

| **Time (min.)** | **0.1 N HCl** | **pH 4.5 acetate buffer** | **pH 5.8 phosphate buffer** | **pH 6.8 phosphate buffer** |
|---|---|---|---|---|
| 0 | 0 | 0 | 0 | 0 |
| 5 | 96 | 58 | 62 | 40 |
| 10 | 100 | 91 | 77 | 51 |
| 15 | 100 | 101 | 82 | 58 |
| 30 | 100 | 104 | 93 | 77 |
| 60 | 100 | 103 | 97 | 94 |

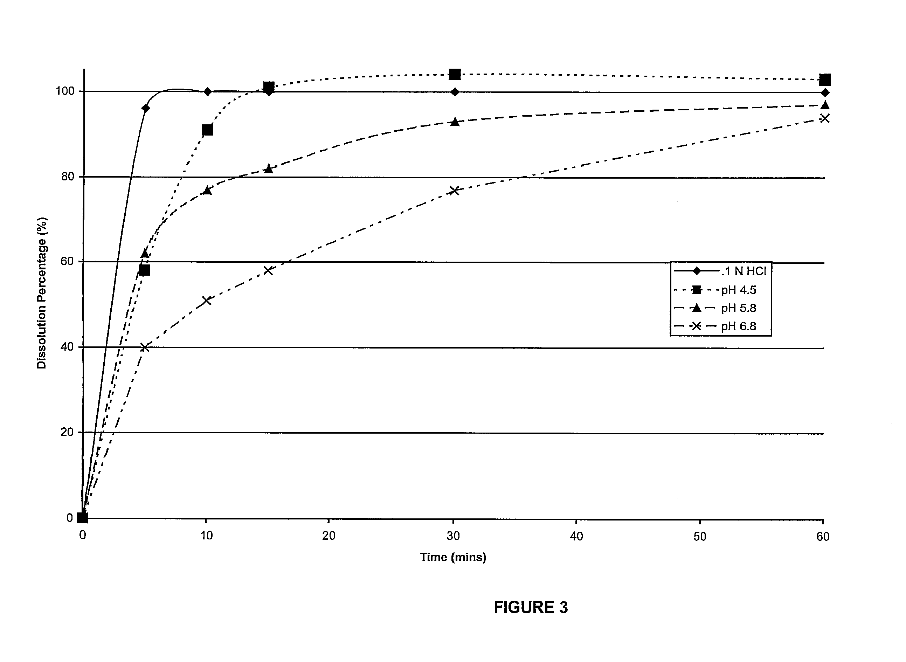

The dissolution profiles of the 10 mg zolpidem FlashDose™ tablet at the various pH values is shown in FIG. 3.

The prior art 10mg Ambien® tablet produced the following dissolution profiles at the various pH values specified in the table below:

| **Time (min.)** | **0.1 N HCl** | **pH 5.8 phosphate buffer** | **pH 6.6 phosphate buffer** | **pH 7.5 phosphate buffer** |
|---|---|---|---|---|
| 0 | 0 | 0 | 0 | 0 |
| 5 | 73 | 96 | 85 | 87 |
| 15 | 103 | 103 | 104 | 94 |
| 30 | 103 | 103 | 106 | 103 |

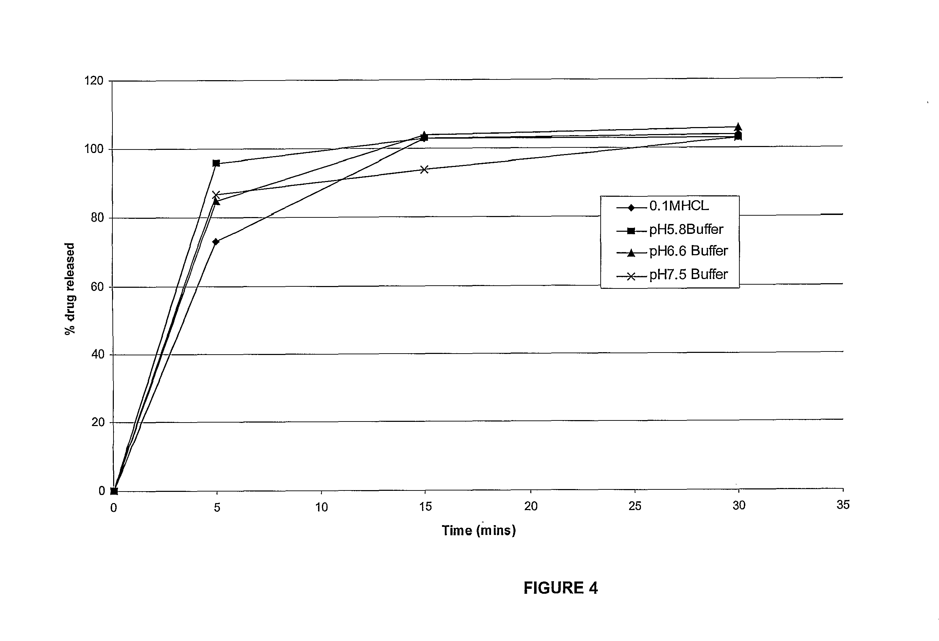

The dissolution profiles of the prior art 10 mg Ambien® tablet at the various pH values is shown in FIG. 4.

Given that the pharmacokinetics of zolpidem tartrate exhibits a food effect, a comparative study was conducted to determine the bioavailability following a single-dose zolpidem tartrate 10 mg FlashDose™ tablet of the invention and the 10 mg prior art Ambien® formulation (Lot OC81). The tablets were dosed at night for both fed (with or after a meal) and fasting conditions. Tables 1 and 2 below summarize the mean plasma zolpidem concentrations (ng/ml) over a 24-hour period under fed and fasted conditions respectively:

| **TABLE 1: FED CONDITIONS** | | |
|---|---|---|
| **Time (Hrs)** | **Zolpidem 10 mg Flash Dose Tablets (ng/ml)** | **Ambien 10 mg Tablets (ng/ml)** |
| 0 | 0.00 ± 0.00 | 0.00 ± 0.00 |
| 0.25 | 17.31 ± 15.70 | 6.02 ± 17.35 |
| 0.5 | 38.08 ± 23.14 | 19.50 ± 35.41 |
| 0.75 | 51.05 ± 23.23 | 29.78 ± 35.62 |
| 1 | 57.70 ± 22.94 | 42.92 ± 41.26 |
| 1.25 | 61.22 ± 22.16 | 47.45 ± 39.96 |
| 1.5 | 63.55 ± 21.25 | 52.89 ± 36.95 |
| 2 | 69.50 ± 23.97 | 61.13 ± 32.81 |
| 2.5 | 72.95 ± 25.04 | 70.11 ± 32.06 |
| 3 | 73.28 ± 26.62 | 74.21 ± 28.72 |
| 3.5 | 71.99 ± 28.37 | 75.92 ± 28.12 |
| 4 | 70.81 ± 30.22 | 75.84 ± 28.88 |
| 6 | 57.47 ± 29.72 | 62.18 ± 29.29 |
| 8 | 39.94 ± 27.11 | 43.12 ± 26.11 |
| 10 | 20.36 ± 16.54 | 21.41 ± 15.86 |
| 12 | 14.63 ± 13.01 | 15.31 ± 11.40 |
| 16 | 6.92 ± 7.14 | 6.77 ± 6.44 |
| 20 | 2.90 ±3.74 | 2.97 ± 3.22 |
| 24 | 1.65 ± 2.37 | 1.58 ± 2.27 |

| **TABLE 2: FASTING STUDY** | | |
|---|---|---|
| **Time (Hrs)** | **Zolpidem 10 mg Flash Dose Tablets** | **Ambien 10 mg Tablets (ng/ml)** |
| 0 | 0.00 ± 0.00 | 0.00 ± 0.00 |
| 0.25 | 12.02 ± 10.42 | 0.68 ± 1.28 |
| 0.5 | 76.40 ± 39.01 | 36.82 ± 41.41 |
| 0.75 | 96.23 ± 43.66 | 62.91 ± 52.90 |
| 1 | 96.57 ± 38.08 | 80.60 ± 52.05 |
| 1.25 | 99.43 ± 37.33 | 91.59 ± 49.59 |
| 1.5 | 97.87 ± 38.21 | 96.66 ± 52.09 |
| 1.75 | 96.10 ± 37.30 | 101.89 ± 48.68 |
| 2 | 94.74 ± 36.20 | 104.21 ± 47.57 |
| 3 | 82.10 ± 34.47 | 91.37 ± 42.62 |
| 4 | 68.41 ± 30.03 | 80.49 ± 40.75 |
| 6 | 49.51 ± 26.40 | 55.51 ± 34.79 |
| 8 | 35.46 ± 26.18 | 39.66 ± 31.68 |
| 10 | 20.75 ± 15.34 | 23.56 ± 22.14 |
| 12 | 15.49 ± 12.46 | 18.60 ± 23.80 |
| 16 | 6.88 ± 6.96 | 8.61 ± 13.88 |
| 20 | 3.31 ± 4.33 | 4.12 ± 7.15 |
| 24 | 1.69 ± 2.85 | 2.28 ± 4.92 |

A comparison of the mean in vivo absorption rate of the zolpidem tartrate 10 mg FlashDose™ tablet of the invention and the 10 mg prior art Ambien® formulation (Lot OC81) can be determined from the data above using the Wagner-Nelson numerical deconvolution method, a statistical method well known in the art and recognized by the US Food and Drug administration. Tables 3 and 4 below summarize the comparison absorption data over the first hour after administration of the two tablets under fed and fasted conditions respectively:

| **TABLE 3: FED STUDY** | | | | | |
|---|---|---|---|---|---|
| **10 mg Zolpidem FlashDose^{™} Tablet** | | | **10 mg Ambien^{®} Tablet** | | |
| **Time (hr)** | **Concentration (ng/ml)** | **% Absorbed** | **Time (hr)** | **Concentration (ng/ml)** | **% Absorbed** |
| 0 | 0.00 | 0.00 | 0 | 0.00 | 0.00 |
| 0.25 | 17.31 | 12.03 | 0.25 | 5.93 | 4.08 |
| 0.5 | 38.08 | 26.99 | 0.5 | 19.5 | 13.58 |
| 0.75 | 51.05 | 37.16 | 0.75 | 29.78 | 21.23 |
| 1 | 57.7 | 43.34 | 1 | 42.3 | 30.73 |
| 1.25 | 61.22 | 47.54 | 1.25 | 47.46 | 35.55 |
| 1.5 | 63.55 | 51.03 | 1.5 | 52.84 | 40.66 |
| 1.75 | 69.54 | 57.12 | 1.75 | 61.13 | 47.95 |
| 2 | 72.95 | 61.61 | 2 | 70.11 | 55.95 |
| 3 | 73.28 | 70.75 | 3 | 75.1 | 67.99 |
| 4 | 71.99 | 78.73 | 4 | 75.92 | 77.58 |
| 6 | 70.81 | 95.33 | 6 | 75.84 | 95.7 |
| 8 | 57.47 | 101.9 | 8 | 62.17 | 103.02 |
| 10 | 39.94 | 101.87 | 10 | 43.12 | 102.81 |
| 12 | 20.36 | 95.91 | 12 | 21.41 | 95.94 |
| 16 | 14.63 | 100.55 | 16 | 15.31 | 100.63 |
| 20 | 6.92 | 100.56 | 20 | 6.77 | 100.17 |
| 24 | 2.66 | 100 | 24 | 3.03 | 100 |

| **TABLE 4: FASTING STUDY** | | | | | |
|---|---|---|---|---|---|
| **10 mg Zolpidem FlashDose^{™} Tablet** | | | **10 mg Ambien^{®} Tablet** | | |
| **Time (hr)** | **Concentration (ng/ml)** | **% Absorbed** | **Time (hr)** | **Concentration (ng/ml)** | **% Absorbed** |
| 0 | 0.00 | 0.00 | 0 | 0.00 | 0.00 |
| 0.25 | 12.02 | 9.38 | 0.25 | 0.68 | 0.53 |
| 0.5 | 74.78 | 58.77 | 0.5 | 36.82 | 28.78 |
| 0.75 | 95.87 | 77.89 | 0.75 | 62.64 | 50.16 |
| 1 | 96.03 | 81.44 | 1 | 79.58 | 65.45 |
| 1.25 | 98.25 | 86.60 | 1.25 | 91.59 | 77.45 |
| 1.5 | 97.87 | 89.81 | 1.5 | 95.52 | 83.52 |
| 1.75 | 95.17 | 91.19 | 1.75 | 101.89 | 91.64 |
| 2 | 94.74 | 94.25 | 2 | 104.21 | 96.80 |
| 3 | 83.97 | 98.79 | 3 | 92.87 | 101.08 |
| 4 | 68.41 | 97.81 | 4 | 80.49 | 103.00 |
| 6 | 49.51 | 100.23 | 6 | 56.93 | 103.05 |
| 8 | 35.46 | 101.64 | 8 | 39.66 | 102.53 |
| 10 | 20.75 | 98.45 | 10 | 23.56 | 98.53 |
| 12 | 16.06 | 100.13 | 12 | 18.60 | 100.28 |
| 16 | 7.07 | 99.88 | 16 | 8.61 | 99.79 |
| 20 | 3.11 | 99.76 | 20 | 4.16 | 99.74 |
| 24 | 1.64 | 100.00 | 24 | 2.28 | 100.00 |

Tables 5 and 6 provide the mean pharmacokinetic parameters for plasma zolpidem after a single-dose zolpidem tartrate 10 mg FlashDose™ tablet of the invention and the 10 mg prior art Ambien^{®} formulation (Lot OC81) under fed and fasting conditions respectively. The tablets were dosed at night for both fed (within 30 minutes of the last meal) and fasting conditions (at least 4 hours after the last meal).

| | **TABLE 5: FED STUDY (n=33)** | | | | | | | | | |
|---|---|---|---|---|---|---|---|---|---|---|
| | **10mg Zolpidem FlashDose^{™} Tablet** | | | | | **10mg Ambien^{®} Tablet** | | | | |
| | **AUC₍₀₋ₜ₎ (ng.hr/ ml)** | **AUC_{(0-inf)} (ng.hr/m I)** | **Cmax (ng/ml )** | **Tₘₐ x (hr)** | **T_{1/2} (hr)** | **AUC₍₀₋ₜ₎ (ng.hr/ml )** | **AUC_{(0-inf)} (ng.hr/m I)** | **Cₘₐₓ (ng/ml )** | **Tmax (hr)** | **T_{1/} 2 (hr )** |
| Mean | 632.6 | 645.9 | 82.4 | 2.8 | 3.0 | 631.3 | 644.0 | 94.1 | 3.2 | 3.1 |
| Std. Dev. | 325.5 | 337.7 | 26.6 | 1.3 | 1.0 | 294.0 | 305.7 | 32.4 | 1.7 | 0.9 |
| CV% | 51.5 | 52.3 | 32.3 | 47. 0 | 34.0 | 46,6 | 47.5 | 34.4 | 51.4 | 30. 3 |
| Min. | 216.1 | 219.7 | 42.0 | 0.5 | 1.4 | 266.8 | 272.9 | 47.4 | 0.5 | 1.5 |
| Max. | 1352.1 | 1407.5 | 141.2 | 6.0 | 4.9 | 1375.2 | 1418.3 | 183.1 | 6.0 | 5.3 |

| | **TABLE 6: FASTING STUDY (n=28)** | | | | | | | | | |
|---|---|---|---|---|---|---|---|---|---|---|
| | **10mg Zolpidem FlashDose^{™} Tablet** | | | | | **10mg Ambien^{®} Tablet** | | | | |
| | **AUC₍₀₋ₜ₎ (ng.hr/ ml)** | **AUC_{(0-inf)} (ng.hr/m I)** | **Cₘₐₓ (ng/ml )** | **Tₘₐ x (hr)** | **T_{1/2} (hr)** | **AUC₍₀₋ₜ₎ (ng.hr/ml )** | **AUC_{(0-inf)} (ng.hr/m I)** | **Cₘₐₓ (ng/ml )** | **Tₘₐₓ (hr)** | **T_{1/2} (hr)** |
| Mean | 688.1 | 702.0 | 112.7 | 1.6 | 3.3 | 741.8 | 778.1 | 121.1 | 1.8 | 3.3 |
| Std. Dev. | 326.2 | 342.2 | 38.2 | 1.0 | 1.0 | 481.2 | 512.6 | 46.6 | 1.0 | 0.9 |
| CV% | 47.4 | 48.7 | 33.9 | 62. 0 | 30.6 | 64.9 | 65.9 | 38.4 | 53.8 | 27.8 |
| Min. | 166.8 | 169.7 | 48.0 | 0.5 | 1.3 | 216.3 | 220.1 | 51.9 | 0.5 | 1.8 |
| Max. | 1764.0 | 1872.7 | 188.9 | 4.0 | 5.9 | 2653.8 | 2830.2 | 238.0 | 4.0 | 5.0 |

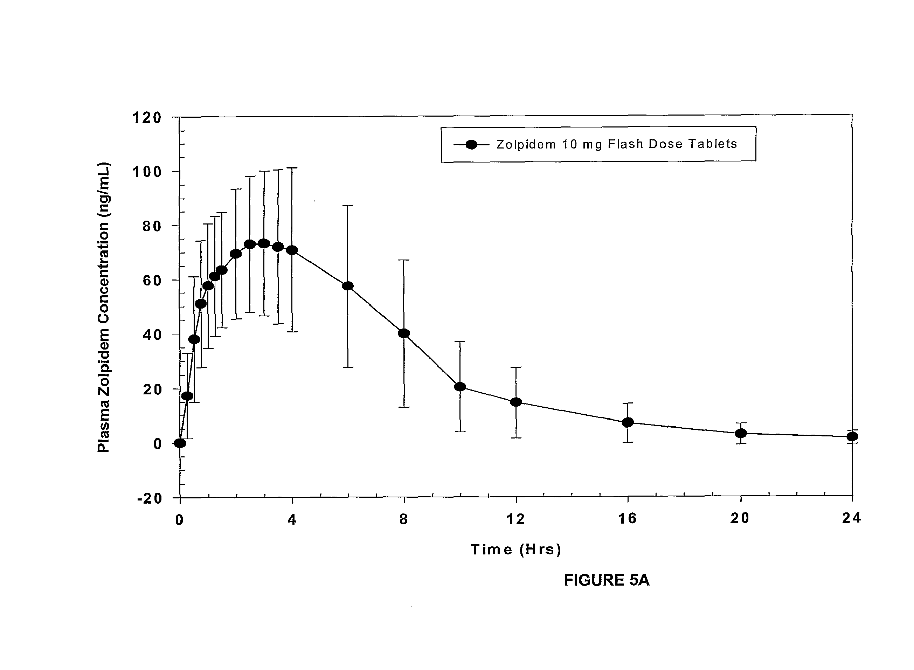

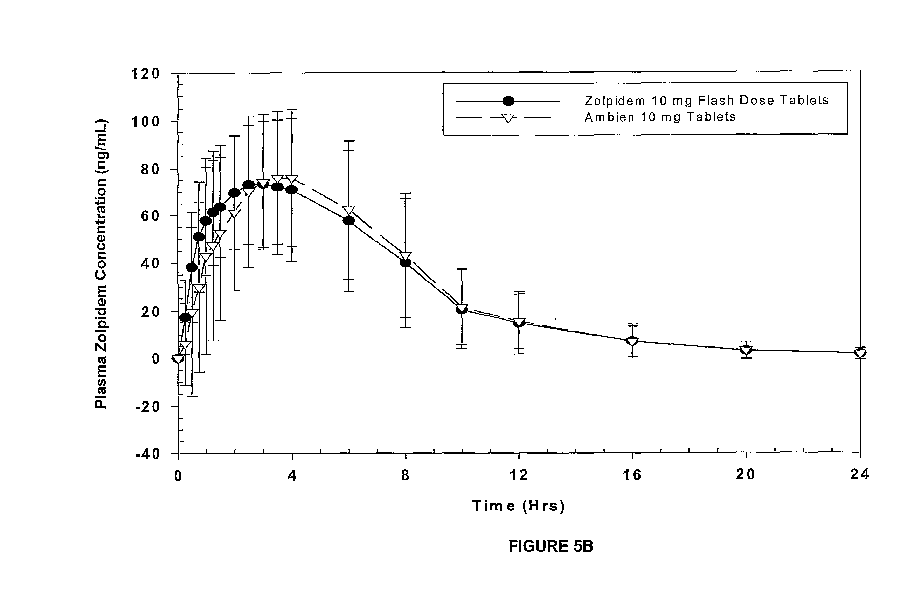

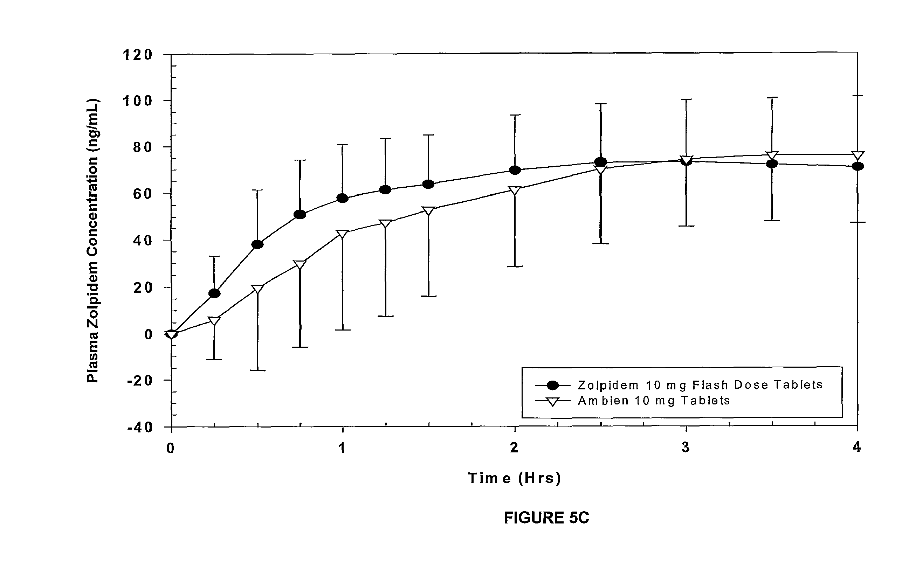

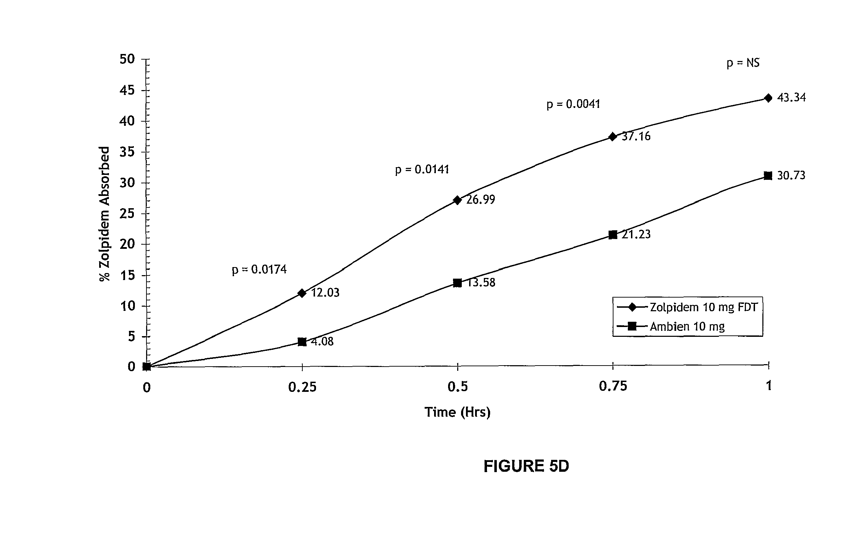

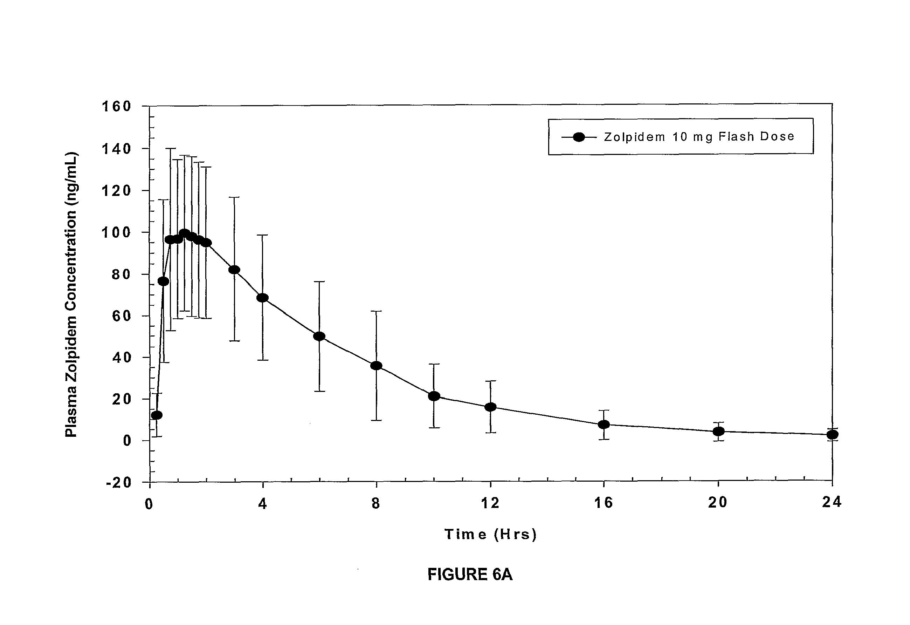

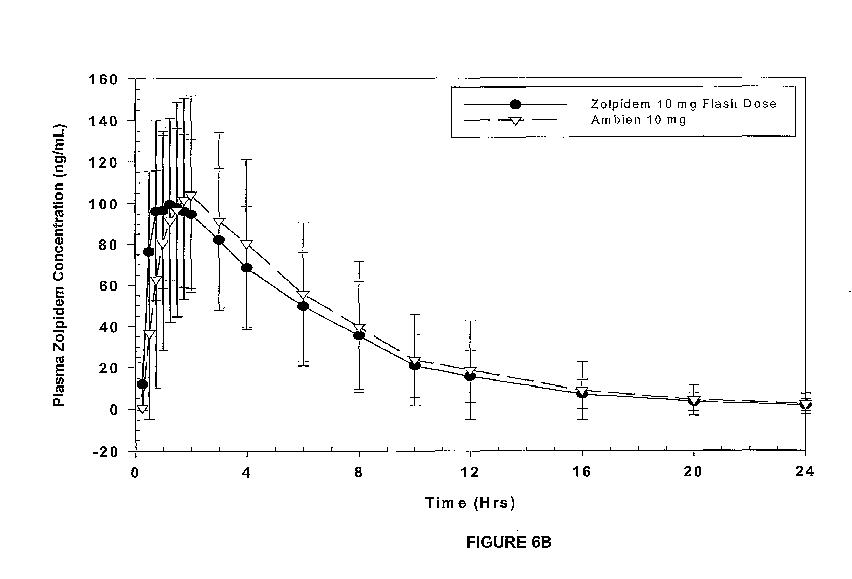

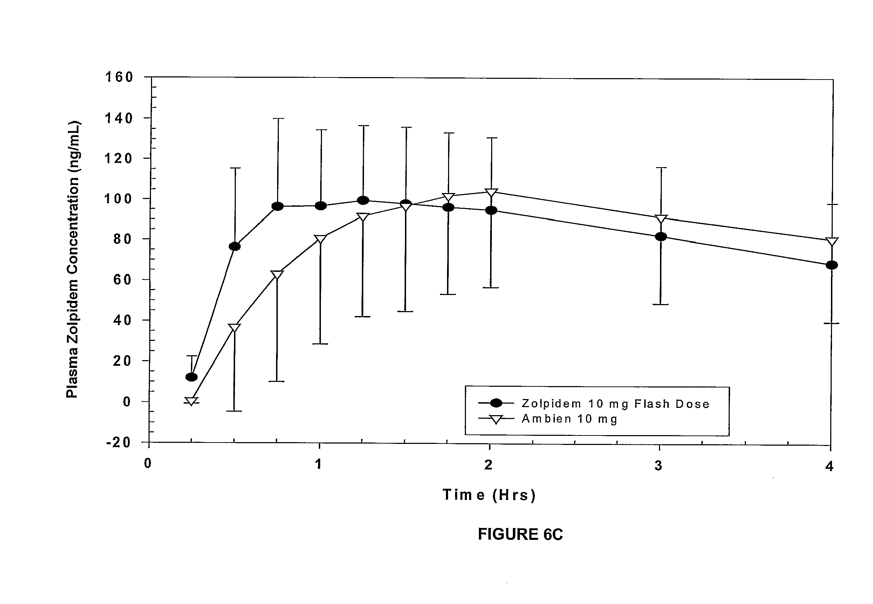

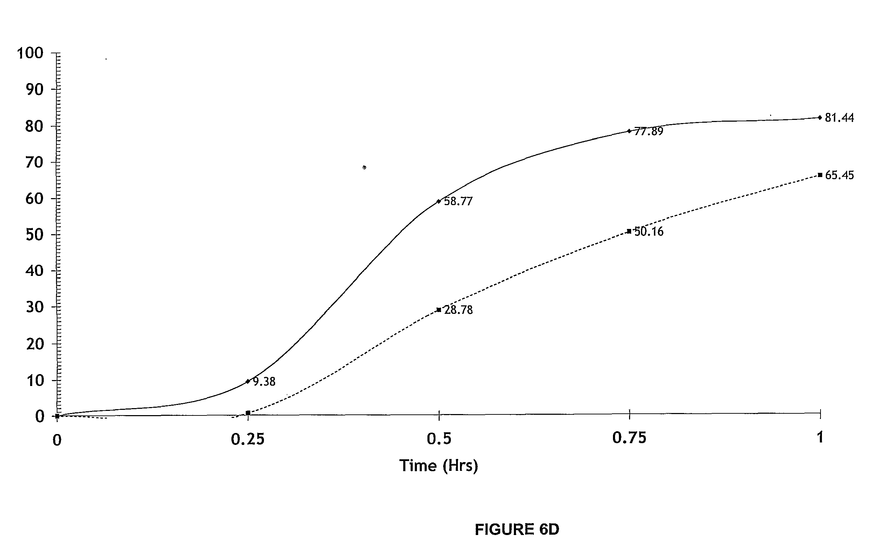

The results reported in the Tables 1-6 and shown in FIGS. 5A-D and FIGS. 6A-D show that there is a significant enhancement in the absorption of the FlashDose™ tablet prepared in accordance with the present invention compared to the prior art 10mg Ambien® formulation in vivo. The absorption profiles generated from deconvolution analysis indicate that there is a significant difference in the in vivo absorption of zolpidem from the FlashDose™ tablet prepared in accordance with the present invention when compared to the prior art Ambien® formulation up to at least the first hour following administration of the tablet in both the fed and fasted state with a greater absorption of zolpidem from the FlashDose™ tablet prepared in accordance with the present invention. ANOVA performed on the concentration at each time point also supports this observation, with statistically significant differences between the FlashDose™ tablet prepared in accordance with the present invention and the prior art Ambien® formulation at 0.25, 0.5 and 0.75 hours (FIG 4D and 5D). These results are particularly surprising given the observation that the in-vitro dissolution of the FlashDose™ tablet prepared in accordance with the present invention is equal or slower at pH 6.8 than the reference marketed zolpidem product Ambien®.

## Claims

1. An enhanced absorption pharmaceutical composition comprising a plurality of microparticles, each microparticle comprising an effective amount of at least one sedative non-benzodiazepine agent, at least one spheronization aid and at least one solubility enhancer.

2. The enhanced absorption pharmaceutical composition of claim **1** wherein said microparticles are about 150 µm to about 500 µm in diameter.

3. The enhanced absorption pharmaceutical composition of claim **2** wherein said microparticles are about 200 µm to about 250 µm.

4. The enhanced absorption pharmaceutical composition of claim **1** wherein said non-benzodiazepine is selected from the group consisting of zolpidem, zaleplon, zoplicone, trazodone, nefazodone, indiplon, esoplicone, chloral hydrate, chloral betaine, mirtazapine, clomethiazole, promethazine, CCD-3693, Co-32693, IP-100-9, PPRT-211, SC-72393, TAK-375, ethychlorvynol, and any combination thereof.

5. The enhanced absorption pharmaceutical composition of claim **4** wherein said non-benzodiazepine is zolpidem.

6. The enhanced absorption pharmaceutical composition of claim **5** wherein said zolpidem is about 1% to about 55% by weight of the microparticle.

7. The enhanced absorption pharmaceutical composition of claim **6** wherein said zolpidem is about 12.5% to about 17.5% by weight of the microparticle.

8. The enhanced absorption pharmaceutical composition of claim **7** wherein said zolpidem is about 15% by weight of the microparticle.

9. The enhanced absorption pharmaceutical composition of claim **1** wherein said spheronization aid is selected from the group consisting of distilled monoglycerides, glyceryl behenate, glyceryl palmitostearate, hydrogenated vegetable oils, polyoxyethylene ethers, cetostearyl alcohol, and any combination thereof.

10. The enhanced absorption pharmaceutical composition of claim **9** wherein said spheronization aid is distilled monoglycerides.

11. The enhanced absorption pharmaceutical composition of claim **10** wherein said distilled monoglycerides is about 5% to about 85% by weight of the microparticle.

12. The enhanced absorption pharmaceutical composition of claim **11** wherein said distilled monoglycerides is about 45% to about 55% by weight of the microparticle.

13. The enhanced absorption pharmaceutical composition of claim **11** wherein said distilled monoglycerides is about 50% by weight of the microparticle.

14. The enhanced absorption pharmaceutical composition of claim **1** wherein said solubility enhancer is selected from the group consisting of macrogol fatty acid esters, poloxamer, polyethylene glycol, polyvinlypyrrolidones, sodium lauryl sulfate, and any combination thereof.

15. The enhanced absorption pharmaceutical composition of claim **14** wherein said solubility enhancer is a macrogol fatty acid ester.

16. The enhanced absorption pharmaceutical composition of claim **15** wherein said macrogol fatty acid ester is from greater than 0% to about 90% by weight of the microparticle.

17. The enhanced absorption pharmaceutical composition of claim **16** wherein said macrogol fatty acid ester is about 30% to about 40% by weight of the microparticle.

18. The enhanced absorption pharmaceutical composition of claim **17** wherein said macrogol fatty acid ester is about 35% by weight of the microparticle.

19. The enhanced absorption pharmaceutical composition of claim **18** wherein said macrogol fatty acid ester is selected from the group consisting of Gelucire 50/13, Gelucire 44/14 and any combination thereof.

20. The enhanced absorption pharmaceutical composition of claim **19** wherein said macrogol fatty acid ester is Gelucire 50/13.

21. The enhanced absorption pharmaceutical composition of claim **1** wherein said microparticles are coated with at least one taste-masking coating.

22. An enhanced absorption pharmaceutical composition comprising a plurality of microparticles, each microparticle comprising an effective amount of zolpidem, distilled monoglycerides and a macrogol fatty acid ester.

23. The enhanced absorption pharmaceutical composition of claim **22** wherein said zolpidem is 15% by weight of the microparticle, said distilled monoglycerides is 50% by weight of the microparticle, and said macrogol fatty acid ester is 35% by weight of the microparticle.

24. The enhanced absorption pharmaceutical composition of claim **23** wherein said macrogol fatty acid ester is selected from the group consisting of Gelucire 50/13, Gelucire 44/14 and any combination thereof

25. The enhanced absorption pharmaceutical composition of claim **24** wherein said macrogol fatty acid ester is Gelucire 50/13.

26. The enhanced absorption pharmaceutical composition of claim **23** wherein said microparticles are coated with at least one taste-masking coating.

27. The enhanced absorption pharmaceutical composition of claim **26** wherein said microparticles are incorporated into a tablet.

28. The enhanced absorption pharmaceutical composition of claim **26** wherein said microparticles are incorporated into a capsule.

29. The enhanced absorption pharmaceutical composition of claim **27** wherein said tablet is an oral fast-dispersing tablet.

30. The enhanced absorption pharmaceutical composition of claim **23** wherein said microparticles are incorporated into a tablet.

31. The enhanced absorption pharmaceutical composition of claim **23** wherein said microparticles are incorporated into a capsule.

32. The enhanced absorption pharmaceutical composition of claim **30** wherein said tablet is an oral fast-dispersing tablet.

33. The use of the enhanced absorption pharmaceutical composition according to claim **1** for the manufacture of a medicament for the treatment of insomnia.

34. The use of the enhanced absorption pharmaceutical composition according to claim **23** for the manufacture of a medicament for the treatment of insomnia.

35. The use of the enhanced absorption pharmaceutical composition according to claim **26** for the manufacture of a medicament for the treatment of insomnia.

36. The use of the enhanced absorption pharmaceutical composition according to claim **1** for the manufacture of an oral fast-dispersing dosage form.

37. The use of the enhanced absorption pharmaceutical composition according to claim **23** for the manufacture of an oral fast-dispersing dosage form.

38. The use of the enhanced absorption pharmaceutical composition according to claim **21** for the manufacture of an oral fast-dispersing dosage form.

39. The use of the enhanced absorption pharmaceutical composition according to claim **26** for the manufacture of an oral fast-dispersing dosage form.

40. An oral fast-dispersing dosage form comprising:
(a) microparticles comprising an effective amount of at least one sedative non-benzodiazepine agent, at least one spheronization aid and at least one solubility enhancer, said microparticles coated with at least one taste-masking coating and adapted for enhanced absorption of the non-benzodiazepine; and
(b) a matrix having enhanced self-binding characteristics;
wherein said coated microparticles are dispersed within said matrix and said dosage form adapted to rapidly dissolve in the mouth of a patient.

41. The oral fast dispersing dosage form of claim **40** wherein said matrix is a shearform matrix consisting essentially of at least one saccharide carrier and at least two sugar alcohols, comprising sorbitol and about 0.5% to about 25% by weight of xylitol which matrix has been treated with at least one crystallization modifier.

42. The oral fast dispersing dosage form of claim **41** wherein said crystallization modifier is Tween 80.

43. The oral fast dispersing dosage form of claim **40** wherein said non-benzodiazepine is selected from the group consisting of zolpidem, zaleplon, zoplicone, trazodone, nefazodone, indiplon, esoplicone, chloral hydrate, chloral betaine, mirtazapine, clomethiazole, promethazine, CCD-3693, Co-32693, IP-100-9, PPRT-211, SC-72393, TAK-375, ethychlorvynol and any combination thereof.

44. The oral fast dispersing dosage form of claim **40** wherein said non-benzodiazepine is zolpidem.

45. The oral fast dispersing dosage form of claim **44** wherein said zolpidem is about 2% to about 12 % by weight of the dosage form.

46. The oral fast dispersing dosage form of claim **45** wherein said zolpidem is about 4% by weight of the dosage form.

47. The oral fast dispersing dosage form of claim **40** wherein said spheronization aid is selected from the group consisting of distilled monoglycerides, glyceryl behenate, glyceryl palmitostearate, hydrogenated vegetable oils, polyoxyethylene ethers, cetostearyl alcohol, and any combination thereof.

48. The oral fast dispersing dosage form of claim **47** wherein said spheronization aid is distilled monoglycerides.

49. The oral fast dispersing dosage form of claim **48** wherein said distilled monoglycerides is about 13.33% by weight of the dosage form.

50. The oral fast dispersing dosage form of claim **40** wherein said solubility enhancer is selected from the group consisting of macrogol fatty acid esters, poloxamer, polyethylene glycol, polyvinylpyrrolidones, sodium lauryl sulfate and any combination thereof.

51. The oral fast dispersing dosage form of claim **50** wherein said solubility enhancer is a macrogol fatty acid ester.

52. The oral fast dispersing dosage form of claim **51** wherein said macrogol fatty acid ester is about 9.33% by weight of the dosage form.

53. The oral fast dispersing dosage form of claim **52** wherein said macrogol fatty acid ester is selected from the group consisting of Gelucire 50/13, Gelucire 44/14 and any combination thereof.

54. The oral fast dispersing dosage form of claim **53** wherein said macrogol fatty acid ester is Gelucire 50/13.

55. The oral fast dispersing dosage form of claim **40** wherein said zolpidem is about 4% by weight of the dosage form, said distilled monoglycerides is about 13.33% by weight of the dosage form, and said macrogol fatty acid ester is about 9.33% by weight of the dosage form.

56. The oral fast dispersing dosage form of claim **55** wherein said macrogol fatty acid ester is selected form the group consisting of Gelucire 50/13, Gelucire 44/14 and any combination thereof.

57. The oral fast dispersing dosage form of claim **56** wherein said macrogol fatty acid ester is Gelucire 50/13.

58. The oral fast dispersing dosage form of claim **57** wherein said dosage form when administered in the evening to a patient in need of such administration exhibits a blood absorption profile such that after about 0.25 hours at least about 10% of the zolpidem is absorbed, after about 0.5 hours at least about 25% of the zolpidem is absorbed; after about 0.75 hours at least about 35% of the zolpidem is absorbed; after about 1 hour at least about 40% of the zolpidem is absorbed, after about 1.5 hours at least about 50% of the zolpidem is absorbed, after about 1.75 hours at least 55% of the zolpidem is absorbed, after about 2 hours at least about 60% of the zolpidem is absorbed, after about 4 hours at least about 75% of the zolpidem is absorbed, and after about 6 hours more than about 90% of the zolpidem is absorbed, into the blood stream of the patient in the fed state.

59. The oral fast dispersing dosage form of claim **57** wherein said dosage form when administered in the evening to a patient in need of such administration exhibits a blood absorption profile such after about 0.25 hours at least about 5% of the zolpidem is absorbed, after about 0.5 hours at least about 55% of the zolpidem is absorbed, after about 0.75 hours at least about 75% of the zolpidem is absorbed, after about 1 hour at least about 80% of the zolpidem is absorbed, after about 1.5 hours at least about 85% of the zolpidem is absorbed, after about 2 hours at least about 90% of the zolpidem is absorbed, and after about 4 hours at least about 97% of the zolpidem is absorbed, into the blood stream of the patient in the fasted state.

60. The oral fast dispersing dosage form of claim **57** wherein said dosage form when administered in the evening to a patient in need of such administration exhibits a mean zolpidem absorption profile as shown in Figure 5D during at least the first hour after administration to the patient in the fed state.

61. The oral fast dispersing dosage form of claim **57** wherein said dosage form when administered in the evening to a patient in need of such administration exhibits a mean zolpidem absorption profile as shown in Figure 6D during at least the first hour after administration to the patient in the fasted state.

62. The oral fast dispersing dosage form of claim **57** wherein said dosage form when administered in the evening to a patient in need of such administration provides a Tₘₐₓ from about 0.5 hours to about 6 hours and a Cₘₐₓ of about 42 ng/ml to about 141 ng/ml zolpidem in the blood after administration of the dosage form to the patient in the fed state.

63. The oral fast dispersing dosage form of claim **57** wherein said dosage form when administered in the evening to a patient in need of such administration provides a mean Tₘₐₓ of about 2.8 hours and a mean Cₘₐₓ of about 82.4 ng/ml zolpidem in the blood after administration of the dosage form to the patient in the fed state.

64. The oral fast dispersing dosage form of claim **57** wherein said dosage form when administered in the evening to a patient in need of such administration exhibits a plasma profile as shown in Figure 5A when said dosage form is administered in the fed state.

65. The oral fast dispersing dosage form of claim **57** wherein said dosage form when administered in the evening to a patient in need of such administration provides a Tₘₐₓ from about 0.5 hours to about 4 hours and a Cₘₐₓ of about 48 ng/ml to about 189 ng/ml zolpidem in the blood after administration of the dosage form to the patient in the fasted state.

66. The oral fast dispersing dosage form of claim **57** wherein said dosage form when administered in the evening to a patient in need of such administration provides a mean Tₘₐₓ of about 1.6 hours and a mean Cₘₐₓ of about 112.7 ng/ml in the blood after administration of the dosage form to the patient in the fasted state.

67. The oral fast dispersing dosage form of claim 57 wherein said dosage when administered in the evening to a patient in need of such administration exhibits a plasma profile as shown in Figure 6A when said dosage form is administered in the fasted state.

68. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration exhibits a blood absorption profile such that after about 0.25 hours at least about 10% of the zolpidem is absorbed, after about 0.5 hours at least about 25% of the zolpidem is absorbed; after about 0.75 hours at least about 35% of the zolpidem is absorbed; after about 1 hour at least about 40% of the zolpidem is absorbed, after about 1.5 hours at least about 50% of the zolpidem is absorbed, after about 1.75 hours at least 55% of the zolpidem is absorbed, after about 2 hours at least about 60% of the zolpidem is absorbed, after about 4 hours at least about 75% of the zolpidem is absorbed, and after about 6 hours more than about 90% of the zolpidem is absorbed, into the blood stream of the patient in the fed state.

69. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration exhibits a blood absorption profile such after about 0.25 hours at least about 5% of the zolpidem is absorbed, after about 0.5 hours at least about 55% of the zolpidem is absorbed, after about 0.75 hours at least about 75% of the zolpidem is absorbed, after about 1 hour at least about 80% of the zolpidem is absorbed, after about 1.5 hours at least about 85% of the zolpidem is absorbed, after about 2 hours at least about 90% of the zolpidem is absorbed, and after about 4 hours at least about 97% of the zolpidem is absorbed, into the blood stream of the patient in the fasted state.

70. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration provides a Tₘₐₓ from about 0.5 hours to about 6 hours, a Cₘₐₓ of about 42 ng/ml to about 141 ng/ml zolpidem and an AUC₍₀₋ₜ₎ of about 216 ng.hr/ml to about 1352 ng.hr/ml in the blood after administration of the dosage form to the patient in the fed state.

71. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration provides a Tₘₐₓ from about 0.5 hours to about 4 hours, a Cₘₐₓ of about 48 ng/ml to about 189 ng/ml zolpidem and an AUC(₀₋ₜ) of about 167 ng.hr/ml to about 1764 ng.hr/ml in the blood after administration of the dosage form to the patient in the fasted state.

72. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration provides a mean Tₘₐₓ from about 2.8 hours, a mean Cₘₐₓ of about 82.4 ng/ml zolpidem and a mean AUC₍₀₋ₜ₎ of about 633 ng.hr/ml in the blood after administration of the dosage form to the patient in the fed state.

73. An oral fast-dispersing dosage form comprising:
(a) microparticles comprising an effective amount of zolpidem, distilled monoglycerides and a macrogol fatty acid ester, wherein said zolpidem is present in an amount of about 4% by weight of the dosage form, said distilled monoglycerides is present in an amount of about 13.33% by weight of the dosage form and said macrogol fatty acid ester is present in an amount of about 9.33% by weight of the dosage form, said microparticles coated with at least one taste-masking coating and adapted for enhanced absorption of zolpidem into the blood stream of a human; and
(b) a shearform matrix having enhanced self binding characteristics and consisting essentially of at least one saccharide carrier and at least two sugar alcohols, comprising sorbitol and about 0.5% to about 25% by weight of xylitol which matrix has been treated with at least one crystallization modifier, wherein said coated microparticles are dispersed within said shearform matrix and said dosage form is adapted to rapidly dissolve in the mouth of a patient, said dosage form when administered in the evening to a patient in need of such administration provides a mean Tₘₐₓ from about 1.6 hours, a mean Cₘₐₓ of about112.7 ng/ml to about 189 ng/ml zolpidem and a mean AUC₍₀₋ₜ₎ of about 688 ng.hr/ml in the blood after administration of the dosage form to the patient in the fasted state.

74. The oral dosage form of claim **57** wherein said dosage form, when administered orally in the evening to a fed patient in need of such administration, provides a plasma concentration time curve with an AUC_{(0-infinity)} ranging from about 220 ng.hr/ml to about 1408 ng.hr/ml.

75. The oral dosage form of claim **57** wherein said dosage form, when administered orally in the evening to a to a fed patient in need of such administration, provides a plasma concentration time curve with a mean AUC_{(0-infinity)} of about 646 ng.hr/ml.

76. The oral dosage form of claim **57** wherein said dosage form, when administered in the evening to a fasting patient in need of such administration, provides a plasma concentration time curve with an AUC_{(0-infinity)} ranging form about 170 ng.hr/ml to about 1873 ng.hr/ml.

77. The oral dosage form of claim **57** wherein said dosage form, when administered in the evening to a fasting patient in need of such administration, provides a plasma concentration time curve with a mean AUC_{(0-infinity)} of about 702 ng.hr/ml.
